# EUROPEAN PATENT SPECIFICATION

(11) **EP 3 747 263 B1**
(45) Date of publication and mention of the grant of the patent: **01.11.2023**
(21) Application number: 20178625.8
(22) Date of filing: 05.06.2020
(51) Int. Cl.: A01H 1/06, C12N 9/10, A01H 1/04, C12Q 1/6895, A01H 6/38, A01H 5/02, A01H 5/12, C12N 15/82

(54) **NEW PLANTS HAVING A WHITE FOLIAGE PHENOTYPE**
NEUE PFLANZEN MIT WEISSEM BLATTPHÄNOTYP
NOUVELLES PLANTES AYANT UN PHÉNOTYPE À FEUILLAGE BLANC

(30) Priority: 05.06.2019 WO PCT/EP2019/064735
(43) Date of publication of application: 09.12.2020
(73) Proprietor: Klemm & Sohn GmbH & Co. KG, 70378 Stuttgart (DE)
(72) Inventor: Vilperte, Vinicius, 30167 Hannover (DE); Debener, Thomas, 31515 Wunstorf (DE); Boehm, Robert, 71364 Winnenden (DE); Dohm, Andrea, 75177 Pforzheim (DE); von Tubeuf, Guido, 70378 Stuttgart (DE); Ulrich, Sander, 70378 Stuttgart (DE)
(74) Representative: Wittop Koning, Tom Hugo

(56) References cited:
- WO-A1-2005/032242
- CN-A- 105 936 898
- US-P- 4 938
- US-P- 7 250
- US-P- 10 160
- HUIFENG LUO ET AL: "Reduced Anthocyanins in Petioles codes for a GST anthocyanin transporter that is essential for the foliage and fruit coloration in strawberry", JOURNAL OF EXPERIMENTAL BOTANY, vol. 69, no. 10, 10 March 2018 (2018-03-10), pages 2595-2608, XP055655616, GB ISSN: 0022-0957, DOI: 10.1093/jxb/ery096
- E. S. LARSEN ET AL: "A carnation anthocyanin mutant is complemented by the glutathione S-transferases encoded by maize Bz2 and petunia An9", PLANT CELL REPORTS, vol. 21, no. 9, 23 April 2003 (2003-04-23) , pages 900-904, XP055655529, Berlin/Heidelberg ISSN: 0721-7714, DOI: 10.1007/s00299-002-0545-x
- DATABASE EMBL [Online] 4 October 2005 (2005-10-04), "CV03095B1G07.f1 CV03-normalized library Euphorbia esula cDNA clone CV03095B1G07.f1 5, mRNA sequence.", XP002796731, retrieved from EBI accession no. EMBL:DV154667 Database accession no. DV154667
- VINICIUS VILPERTE ET AL: "Hybrid de novo transcriptome assembly of poinsettia (Euphorbia pulcherrima Willd. Ex Klotsch) bracts", BMC GENOMICS, vol. 20, no. 1, 27 November 2019 (2019-11-27), XP055655538, DOI: 10.1186/s12864-019-6247-3
- DATABASE UniProt [Online] 5 December 2018 (2018-12-05), "RecName: Full=Glutathione transferase {ECO:0000256|ARBA:ARBA00012452}; EC=2.5.1.18 {ECO:0000256|ARBA:ARBA00012452};", retrieved from EBI accession no. UNIPROT:A0A2R6R622 Database accession no. A0A2R6R622

## Description

The invention relates to a method for the generation of *Euphorbia pulcherrima* (Poinsettia, also known as Christmas Star) having a white foliage phenotype, to a cultivated *Euphorbia pulcherrima* having a white foliage phenotype, to seeds, plant parts or plant cells derived from *Euphorbia pulcherrima,* to a molecular marker capable of identifying a dysfunctional glutathione S-transferase (GST) gene, to isolated DNA encoding such a dysfunctional GST gene and to the use of such DNA for the preparation of a molecular marker and for use in methods of targeted mutagenesis to inactivate the EpGST gene to create Euphorbia plants with a white foliage phenotype.

Breeding of new plant varieties requires the continuous development of genetic diversity to obtain new, improved characteristics and traits. New genetic diversity can be established by crossing, random mutagenesis, or with the help of modern biotechnology.

While random mutagenesis (e.g., by irradiation or chemical treatment) is efficient for dominant characteristics, it is inefficient for recessive traits if the selection of the outcome only relies on the desired phenotype. To achieve the same change in all alleles of the target plant is statistically highly unlikely, even in a diploid genome. A success is only likely if one of the target alleles is already mutated (e.g., by natural mutation) and the starting plant is therefore already heterozygous with respect to the target gene. Here the desired phenotype can be obtained with reasonable likelihood in a one-step mutagenesis process. However, since for a dominant trait the homozygous and heterozygous plants cannot be distinguished by their phenotypes, identifying the heterozygote is very cumbersome as the nature of the mutation is normally unknown and molecular markers not available. This limits the source material and especially denies the use of high-performing elite inbred lines. Furthermore, it requires complex crossing experiments to identify plants with heterozygous traits and combine them into a homozygous line. A better likelihood is possible with targeted mutagenesis if the target gene is known. This however not only requires a deep understanding of the often complex genetics, but also raises regulatory challenges as the resulting plant may be considered "genetically modified" in some legislations.

Glutathione S-transferase (GST) is described to play a role in foliage and fruit coloration in strawberry, where a premature stop codon in the GST gene results in a white fruit variety (Luo et al., J. Exp. Bot. 2018, vol 69, no 10, pp 2595-2608). GST is also described to complement pale pink maize and Petunia mutants resulting in local darker spots in the petals (Larsen et al., Plant Cell rep. 2003, vol 21, pp. 900-904). The gene sequence of some GST genes has been identified and described e.g. for *Camellia sinensis* in CN105936898A and for *Euphorbia* in the EMBL database (accession no DV154667).

The present inventors have now found that a white foliage phenotype can be obtained in *Euphorbia pulcherrima* in a controlled and reproducible manner. This trait was found to be recessive, for which disablement of a glutathione S-transferase function in the plant appeared to be responsible. The white foliage can be only partial, and the intensity thereof can be dependent on the regime and duration of daylight exposure. The said phenotype appears as white-coloured bracts. Poinsettia is a commercially important plant species of the diverse spurge family (*Euphorbiaceae*)*.* Poinsettia is particularly well known for its red and green foliage and is widely used in Christmas floral displays. The coloured bracts, which are most often flaming red but can be orange, pale green, cream, pink, white, or marbled, are often mistaken for flower petals because of their groupings and colours but are actually leaves. The colouration of the bracts is triggered by photoperiodism, meaning that they require short day conditions (12 hours at a time for at least five days in a row) to change colour. At the same time, the plants require abundant light during the day for the brightest colour.

Poinsettia varieties with white-coloured bracts are known, such as the cultivars Bravo White (Dümmen Orange), Titan White (Syngenta), Christmas Feelings Pearl (Klemm + Sohn), Wintersun (Klemm + Sohn) or those as described in US-PP4,938, US-PP7,250 and US-PP10,160. However, these are either mutants from heterozygous red-coloured varieties and were obtained by chance or are resulting from cumbersome crossing schemes. It has not been possible so far to convert a homozygous red-coloured elite Poinsettia variety into a white-coloured one while retaining their elite properties (i.e., without any further crossing and back-crossing) as the molecular reason for the white-coloured bracts phenotype was not known.

It was now found that a specific mutation in a GST gene family results in a white foliage phenotype and is related to a homozygous knock-out of a gene coding for a Glutathione S-transferase (hereinafter EpGST) protein of SEQ ID No 3, or coding for a functional variant of said protein with at least 60%, preferably 70% or 80%, more 90%, most preferably 95% amino acid identity, the EpGST gene comprising, in the region encoding amino acids at positions 40 - 50 of the protein of SEQ ID No 3, a stretch of 12 nucleotides consisting of a threefold CTTC repeat. It was found that in Poinsettia, a specific CTTC deletion in the said threefold repeat was responsible for the white foliage trait. It is to be observed that the GST genes known so far do not have the decisive stretch of 12 nucleotides consisting of the threefold CTTC repeat. Without intending to be bound to a specific mechanism, surprisingly it has been found that this deletion can be triggered in a reproducible way by using methods of random mutagenesis.

In Poinsettia, the presence of a single functional EpGST gene is now observed to result in coloured i.e. non-white foliage, (in particular red and/or pink coloured foliage).

White-coloured interspecific hybrids of *Euphorbia pulcherrima* and *Euphorbia cornastra,* - being known as Princettia - are described which have been obtained by random mutagenesis (e.g., as described in WO2005/032242). However, the white-coloured bract phenotype of these varieties is based on a different mode of action, as the inventors observed that Princettia with white foliage appear to comprise at least one functional EpGST gene.

In this respect it is noted that the inventors observed that the Poinsettia varieties Alaska and Alpina, both having an extreme bright white foliage (RHS colour code about 149B and about 154C, respectively), i.e. without any substantial shades of yellow, still comprise a functional EpGST in their genome, indicating that for these varieties, another mode of action occurs with regard to the white foliage phenotype. It can e.g. be that these varieties have a mutation in the carotenoid producing pathway, explaining their bright white phenotype.

It was observed that in plants, being homozygous for the mutant EpGST gene, and having the white phenotype of the invention, the white colour of the foliage is not limited to pure white or bright white as observed for Alaska and Alpina, but encompasses off-white variations, in particular some yellowish shading and creamy white shading. The mutant plants as described herein usually have an off-white yellowish shading as compared to the Alaska and Alpina varieties. Without the desire to be bound to any explanation, it is believed that the off-white phenotype for homozygous mutants of the EpGST gene is often not as bright as that of Alaska and Alpina, as in the EpGST mutants the carotenoids, responsible for the foliage colouring is still produced, while this may not be the case for the Alaska and Alpina varieties.

### DEFINITIONS

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques and/or substitutions of equivalent techniques that would be apparent to one of skill in the art.

Following long-standing patent law convention, the terms "a," "an," and "the" refer to "one or more" when used in this application, including the claims. For example, the phrase "a cell" refers to one or more cells, and in some embodiments can refer to a tissue and/or an organ. Similarly, the phrase "at least one", when employed herein to refer to an entity, refers to, for example, 1, 2, 3, 4, 5, 6, 7, 8, 9, 10, 15, 20, 25, 30, 35, 40, 45, 50, 75, 100, or more of that entity, including but not limited to all whole number values between 1 and 100 as well as whole numbers greater than 100.

Unless otherwise indicated, all numbers expressing quantities of ingredients, reaction conditions, and so forth used in the specification and claims are to be understood as being modified in all instances by the term "about." The term "about," as used herein when referring to a measurable value such as an amount of mass, weight, time, volume, concentration or percentage is meant to encompass variations of in some embodiments ± 20%, in some embodiments ± 10%, in some embodiments ± 5%, in some embodiments ±1%, in some embodiments ± 0.5%, and in some embodiments ± 0.1% from the specified amount, as such variations are appropriate to perform the disclosed methods and/or employ the discloses compositions, nucleic acids, polypeptides, etc. Accordingly, unless indicated to the contrary, the numerical parameters set forth in this specification and attached claims are approximations that can vary depending upon the desired properties sought to be obtained by the presently disclosed subject matter.

As used herein, the term "allele" refers to a variant or an alternative sequence form at a genetic locus. In diploids such as Poinsettia, a single allele is inherited by a progeny individual separately from each parent at each locus. The two alleles of a given locus present in a diploid organism occupy corresponding places on a pair of homologous chromosomes, although one of ordinary skill in the art understands that the alleles in any particular individual do not necessarily represent all of the alleles that are present in the species.

As used herein, the term "and/or" when used in the context of a list of entities, refers to the entities being present singly or in combination. Thus, for example, the phrase "A, B, C, and/or D" includes A, B, C, and D individually, but also includes any and all combinations and subcombinations of A, B, C, and D (e.g., AB, AC, AD, BC, BD, CD, ABC, ABD, and BCD). In some embodiments, one of more of the elements to which the "and/or" refers can also individually be present in single or multiple occurrences in the combinations(s) and/or subcombi nation(s).

As used herein, the phrase "associated with" refers to a recognizable and/or assayable relationship between two entities. For example, a marker is "associated with" a trait when it is linked to it and when the presence of the marker is an indicator of whether and/or to what extent the desired trait or trait form will occur in a plant/germplasm comprising the marker. Similarly, a marker is "associated with" an allele when it is linked to it and when the presence of the marker is an indicator of whether the allele is present in a plant/germplasm comprising the marker. For example, "a marker associated with EpGST gene" or the "CTTC deletion" refers to a marker whose presence or absence can be used to predict whether a plant is homozygote or heterozygote for the functional or dysfunctional EpGST gene.

The term "comprising," which is synonymous with "including," "containing," and "characterized by," is inclusive or open-ended and does not exclude additional, unrecited elements and/or method steps. "Comprising" is a term of art that means that the named elements and/or steps are present, but that other elements and/or steps can be added and still fall within the scope of the relevant subject matter.

As used herein, the phrase "consisting of" excludes any element, step, or ingredient not specifically recited. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of the related disclosure or claim to the specified materials and/or steps, plus those that do not materially affect the basic and novel characteristic(s) of the disclosed and/or claimed subject matter.

With respect to the terms "comprising," "consisting essentially of," and "consisting of," where one of these three terms is used herein, the presently disclosed and claimed subject matter can include in some embodiments the use of either of the other two terms. For example, if a subject matter relates in some embodiments to nucleic acids that encode polypeptides comprising amino acid sequences that are at least 60%, preferably 70% or 80%, more 90%, most preferably 95% identical to a SEQ ID NO: 3, It is understood that the disclosed subject matter thus also encompasses nucleic acids that encode polypeptides that in some embodiments consist essentially of amino acid sequences that are at least 60%, preferably 70% or 80%, more 90%, most preferably 95% identical to that SEQ ID NO: 3 as well as nucleic acids that encode polypeptides that in some embodiments consist of amino acid sequences that are at least 60%, preferably 70% or 80%, more 90%, most preferably 95% identical to that SEQ ID NO: 3. Similarly, it is also understood that in some embodiments the methods for the disclosed subject matter comprise the steps that are disclosed herein, in some embodiments the methods for the presently disclosed subject matter consist essentially of the steps that are disclosed, and in some embodiments the methods for the presently disclosed subject matter consist of the steps that are disclosed herein.

As used herein, the term "gene" refers to a hereditary unit including a sequence of DNA that occupies a specific location on a chromosome and that contains the genetic instruction for a particular characteristic or trait in an organism.

A "genetic map" is a description of genetic linkage relationships among loci on one or more chromosomes within a given species, generally depicted in a diagrammatic or tabular form.

As used herein, the term "human-induced mutation" refers to any mutation that occurs as a result of either direct or indirect human action. This term includes, but is not limited to, mutations obtained by any method of targeted or human-induced random mutagenesis.

As used herein, "introduced" means delivered, expressed, applied, transported, transferred, permeated, or other like term to indicate the delivery, whether of nucleic acid or protein or combination thereof, of a desired object to an object.

As used herein, the terms "marker", "marker probe" and "probe" refer to a nucleotide sequence or nucleic acid molecule that can be used to detect the presence or absence of a sequence within a larger sequence, e.g., a nucleic acid probe that is complementary to all of or a portion of the marker or marker locus, through nucleic acid hybridization. Marker probes comprising about 8, 10, 15, 20, 30, 40, 50, 60, 70, 80, 90, 100 or more contiguous nucleotides can be used for nucleic acid hybridization.

As used herein, the term "molecular marker" can be used to refer to a genetic marker, as defined above, or an encoded product thereof (e.g., a protein) used as a point of reference when identifying the presence/absence of a CTTC deletion in a EpGST gene. A molecular marker can be derived from genomic nucleotide sequences or from expressed nucleotide sequences (e.g., from a RNA, a cDNA, etc.). The term also refers to nucleotide sequences complementary to or flanking the marker sequences, such as nucleotide sequences used as probes and/or primers capable of amplifying the marker sequence. Nucleotide sequences are "complementary" when they specifically hybridize in solution (e.g., according to Watson-Crick base pairing rules). This term also refers to the genetic markers that indicate a trait by the absence of the nucleotide sequences complementary to or flanking the marker sequences, such as nucleotide sequences used as probes and/or primers capable of amplifying the marker sequence.

As used herein, the terms "nucleotide sequence," "polynucleotide," "nucleic acid sequence," "nucleic acid molecule," and "nucleic acid fragment" refer to a polymer of RNA or DNA that is single- or double-stranded, optionally containing synthetic, non-natural, and/or altered nucleotide bases. A "nucleotide" is a monomeric unit from which DNA or RNA polymers are constructed and consists of a purine or pyrimidine base, a pentose, and a phosphoric acid group. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

As used herein, the term "nucleotide sequence identity" refers to the presence of identical nucleotides at corresponding positions of two polynucleotides. Polynucleotides have "identical" sequences if the sequence of nucleotides in the two polynucleotides is the same when aligned for maximum correspondence (e.g., in a comparison window). Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence identity" for polynucleotides, such as about 50, 55, 60, 65, 70, 75, 80, 85, 90, 95, 98, 99 or 100 percent sequence identity, can be determined by comparing two optimally aligned sequences over a comparison window, wherein the portion of the polynucleotide sequence in the comparison window can include additions or deletions (i.e., gaps) as compared to the reference sequence for optimal alignment of the two sequences. In some embodiments, the percentage is calculated by: (a) determining the number of positions at which the identical nucleic acid base occurs in both sequences; (b) dividing the number of matched positions by the total number of positions in the window of comparison; and (c) multiplying the result by 100. Optimal alignment of sequences for comparison can also be conducted by computerized implementations of known algorithms, or by visual inspection. Readily available sequence comparison and multiple sequence alignment algorithms are, respectively, the Basic Local Alignment Search Tool (BLAST) and ClustalW/ClustalW2/Clustal Omega programs available on the Internet (e.g., the website of the EMBL-EBI). Other suitable programs include, but are not limited to, GAP, BestFit, Plot Similarity, and FASTA, which are part of the Accelrys GCG Package available from Accelrys, Inc. of San Diego, Calif., United States of America. See also Smith & Waterman, 1981; Needleman & Wunsch, 1970; Pearson & Lipman, 1988; Ausubel et al., 1988; and Sambrook & Russell, 2001.

One example of an algorithm that is suitable for determining percent sequence identity and sequence similarity is the BLAST algorithm, which is described in Altschul et al., 1990. In some embodiments, a percentage of sequence identity refers to sequence identity over the full length of one of the gDNA, cDNA, or the predicted protein sequences in the largest ORF of SEQ ID No: 1 or 61 being compared. In some embodiments, a calculation to determine a percentage of nucleic acid sequence identity does not include in the calculation any nucleotide positions in which either of the compared nucleic acids includes an "N" (i.e., where any nucleotide could be present at that position).

The term "open reading frame" (ORF) refers to a nucleic acid sequence that encodes a polypeptide. In some embodiments, an ORF comprises a translation initiation codon (i.e., start codon), a translation termination (i.e., stop codon), and the nucleic acid sequence there between that encodes the amino acids present in the polypeptide. The terms "initiation codon" and "termination codon" refer to a unit of three adjacent nucleotides (i.e., a codon) in a coding sequence that specifies initiation and chain termination, respectively, of protein synthesis (mRNA translation).

As used herein, the terms "phenotype," "phenotypic trait" or "trait" refer to one or more traits of a plant or plant cell. The phenotype can be observable to the naked eye, or by any other means of evaluation known in the art, e.g., microscopy, biochemical analysis, or an electromechanical assay. In some cases, a phenotype is directly controlled by a single gene or genetic locus (i.e., corresponds to a "single gene trait").

As used herein, the term "plant" can refer to a whole plant, any part thereof, or a cell or tissue culture derived from a plant. Thus, the term "plant" can refer to any of whole plants, plant components or organs (e.g., leaves, stems, roots, etc.), plant tissues, seeds and/or plant cells.

A plant cell is a cell of a plant, taken from a plant, or derived through culture from a cell taken from a plant. Thus, the term "plant cell" includes without limitation cells within seeds, suspension cultures, embryos, meristematic regions, callus tissue, leaves, shoots, gametophytes, sporophytes, pollen, and microspores. The phrase "plant part" refers to a part of a plant, including single cells and cell tissues such as plant cells that are intact in plants, cell clumps, and tissue cultures from which plants can be regenerated. Examples of plant parts include, but are not limited to, single cells and tissues from pollen, ovules, leaves, embryos, roots, root tips, anthers, flowers, fruits, stems, shoots, and seeds; as well as scions, rootstocks, protoplasts, calli, and the like.

As used herein, the term "primer" refers to an oligonucleotide which is capable of annealing to a nucleic acid target (in some embodiments, annealing specifically to a nucleic acid target) allowing a DNA polymerase and/or reverse transcriptase to attach thereto, thereby serving as a point of initiation of DNA synthesis when placed under conditions in which synthesis of a primer extension product is induced (e.g., in the presence of nucleotides and an agent for polymerization such as DNA polymerase and at a suitable temperature and pH). In some embodiments, one or more pluralities of primers are employed to amplify plant nucleic acids (e.g., using the polymerase chain reaction; PCR).

As used herein, the term "probe" refers to a nucleic acid (e.g., a single stranded nucleic acid or a strand of a double stranded or higher order nucleic acid, or a subsequence thereof) that can form a hydrogen-bonded duplex with a complementary sequence in a target nucleic acid sequence. Typically, a probe is of sufficient length to form a stable and sequence-specific duplex molecule with its complement, and as such can be employed in some embodiments to detect a sequence of interest present in a plurality of nucleic acids.

As used herein, the terms "progeny" and "progeny plant" refer to a plant generated from vegetative or sexual reproduction from one or more parent plants. A progeny plant can be obtained by cloning or selfing a single parent plant, or by crossing two or more parental plants. For instance, a progeny plant can be obtained by cloning or selfing of a parent plant or by crossing two parental plants and include selfings as well as the F1 or F2 or still further generations. An F1 is a first-generation progeny produced from parents at least one of which is used for the first time as donor of a trait, while progeny of second generation (F2) or subsequent generations (F3, F4, and the like) are specimens produced from selfings, intercrosses, backcrosses, and/or other crosses of F1s, F2s, and the like.

As used herein, the term "reference sequence" refers to a defined nucleotide sequence used as a basis for nucleotide sequence comparison. In some embodiments, any of SEQ ID NOs: 1, 2 and 61 can serve as a reference sequence for comparing to other sequences obtained from plants.

As used herein, the term "regenerate," and grammatical variants thereof, refers to the production of a plant from tissue culture.

As used herein, the term "trait" refers to a phenotype of interest, a gene that contributes to a phenotype of interest, as well as a nucleic acid sequence associated with a gene that contributes to a phenotype of interest.

As used herein, the term , the term "human-induced random mutagenesis" is intended to be understood in a conventional manner, i.e., to indicate an introduction of one or more mutations at random positions of the target sequence (i.e., as opposed to site-specific mutagenesis). The random mutations are typically introduced by exposing a DNA sequence to be modified to a mutagen and then screening for the presence of variants. The plant or part thereof that is exposed to the mutagen may be any part of the plant from which a mutated plant can be generated. For example, the plant or part of a plant may be the entire plant or a cutting, a shoot, a seed, an embryo, an ovule, a bract, a leaf or any other part of the plant from which a mutated plant can be generated. The random mutagenesis of the DNA sequence may conveniently be performed by use of any method known in the art. For instance, the random mutagenesis may be performed by use of a suitable physical or chemical mutagenizing agent, by use of any combination of these mutagenizing agents. Preferred a agents which cause a DNA double-strand break.

As used herein, the term "mutagen" refers to a compound or process that results in the introduction of mutations in the plant genome. Examples of suitable mutagens include biological mutagens such as transposons, chemical mutagens such as N-nitroso-N-ethylurea, N-nitroso-N-methylurea, ethylmethane sulfonate (EMS), sodium azide, radiation, or other mutagens. The radiation may be ultra-violet radiation, X-ray radiation, gamma radiation, alpha radiation, beta radiation, ion beams such as 4He²⁺ and H⁺, etc. Preferably, the radiation is gamma radiation or UV radiation. The dose of gamma radiation will vary depending on the target plant, the size of the plant or part thereof and the robustness of the target plant. Preferably, the dose of gamma radiation is between 1 and 10,000 rads of gamma radiation. Preferably, the dose is between 1000 and 10000 rads. More preferably, the dose is between 2,000 and 8,000 rads.

As used herein, the term "targeted mutagenesis" or "mutagenesis strategy" refers to any method of mutagenesis that results in the intentional mutagenesis of a chosen gene. Targeted mutagenesis includes the methods CRISPR, TILLING, TALEN, and other methods not yet discovered but which may be used to achieve the same outcome.

As used herein, the term 'white' with respect to the white foliage phenotype of the Poinsettia of the invention is not limited to pure white or bright white, but encompasses off-white variation, in particular some yellowish shading and creamy white shading. The skilled person will immediately recognize the white foliage of the invention as 'white', as the term off-white still indicates that main colour as experience by the observed is white. E.g. white foliage having a yellowish shade is still of white colour and be regarded and experienced as such by the skilled person and any interested observant.

GSTs comprise a family of eukaryotic and prokaryotic metabolic enzymes best known for their ability to catalyse the conjugation of the reduced form of glutathione (GSH) to xenobiotic substrates. It was found that GST plays a role in sequestering of the red pigment anthocyanin into the vacuole, which is an essential step for the expression of the red colour. The knock-out of the transport mechanism provides a clear white foliage phenotype and provides a new mode of action to create white flowering plants in plants that express the EpGST as defined above.

The EpGST gene is herein defined as encoding an enzyme having the amino acid sequence of SEQ ID No 3 or encoding a functional homolog or variant of said protein with at least 60%, preferably 70% or 80%, more 90%, most preferably 95% amino acid identity, the gene having the 12 nucleotides stretch [CTTC]₃ in the region encoding amino acids at positions 40 - 50 of the protein. The said homolog or variant preferably having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, as well as the 12 nucleotides stretch [CTTC]₃ in the region encoding amino acids at positions 40 - 50 of the protein.

The protein has glutathione S-transferase activity, i.e. capable of catalysing the conjugation of the reduced form of glutathione (GSH) to xenobiotic substrates, in particular a cytosolic GST enzyme, more in particular a GST enzyme capable of sequestering of the red pigment anthocyanin into the vacuole. The GST enzyme encoded by the gene defined herein is in an attractive embodiment defined by having a flavonoid binding affinity, i.e. substrate specificity at the N-terminus of the protein. This functionality or dysfunctionality can be determined in a biochemical assay such as a ligand fishing assay (Dixon & Edwards (2010) J. Biol. Chem. 285, 36322-36329). In particular, a GST enzyme that binds to an anthocyanin affinity column can be regarded as being a functional enzyme. To this end, a protein mixture from a plant extract can be passed over an affinity matrix comprising anthocyanins (or precursors thereof) immobilized thereon, and functional GST enzyme molecules bind to the said ligands and can be eluted afterwards, if desired. Dysfunctional GST enzymes will not bind or bind to a significantly lesser extent.

The dysfunctionality can also be assessed by a genetic complementation of mutant lines (Li et al. (2011) Plant Cell Environ. 34, 374-388, Sun et al. (2012) Mol. Plant 5, 387-400, Alfenito et al. (1998) Plant Cell 10, 1135-1149, and Smith et al. (2003) Plant J. 36, 433-442. Dysfunctional GST enzymes will preferably not bind to an anthocyanin column but binding of less than 60% of binding by the wild type EpGST enzyme, i.e. as depicted in SEQ ID No 3, can already result in loss of foliage colouration and can therefore be regarded as dysfunctional. More preferably the binding will be less than 50%, 40%, 30%, 20% or 10% than that of the wild type GST enzyme. A functional variant or homolog of the GST enzyme will bind with an affinity of at least 60%, preferably of at least 70%, more preferably of at least 80% and even more preferably of at least 90 % or more of the binding affinity of the wild type GST enzyme. In another embodiment, the dysfunctionality may also affect the GST capability as a transport protein.

Accordingly, the glutathione S-transferase EpGST cDNA has SEQ ID No 1 encoding the protein of SEQ ID No 3, or encodes a functional homolog or variant thereof with at least 60% (or 70, 75, 80, 85, 90, 91, 92, 93, 94, 95, 96, 97, 98 or 99%) amino acid identity, the said homolog or variant preferably having a first domain at positions 11 - 13 of SEQ ID No 3 being AAC, AGC or AAN, where N can be any amino acid, a second domain at positions 53 - 56 of SEQ ID No 3 being LVPA, QVPA or QVPV, and a third amino acid domain at positions 65 - 68 of SEQ ID No 3 being FESR. In this respect, a functional homolog is to be understood as a gene encoding a protein having the given amino acid identity and capable of conferring white foliage phenotype to the plant when present in a homozygous fashion. The three domains indicated above have been identified earlier (Conn et al., (2008) J. Exp. Bot. 59(13), 3621-3634 and found to play an important and decisive role in anthocyanin binding. The said EpGST gene is found in Euphorbia, in particular in Poinsettia.

By the present invention, a simplified and predictable method is provided to establish a white-foliage phenotype in plants, that express the above EpGST, such as Poinsettia.

To this end, the invention provides a method for the generation of a *Euphorbia pulcherrima (*Poinsettia*)* plant having a white foliage phenotype, comprising the steps of:
a. providing a *Euphorbia pulcherrima (*Poinsettia*)* plant without a white foliage phenotype comprising in its genome at least one functional allele of a glutathione S-transferase gene EpGST encoding a protein of SEQ ID No 3 or a functional variant of said protein with at least 90%, preferably 95% amino acid identity, the EpGST gene comprising, in the region encoding amino acids at positions 40 - 50 of the protein of SEQ ID No 3, a stretch of 12 nucleotides consisting of a threefold CTTC repeat, wherein the *Euphorbia pulcherrima* (Poinsettia) plant is heterozygous for the EpGST gene, comprising one functional allele as well as one dysfunctional allele comprising the CTTC deletion, said heterozygous *Euphorbia pulcherrima (*Poinsettia*)* plant without a white foliage phenotype being selected by a molecular marker suitable for the detection of the CTTC deletion;;
b. subjecting the *Euphorbia pulcherrima (*Poinsettia*)* plant of step a. to a mutagenesis treatment to produce a mutant *Euphorbia pulcherrima* (Poinsettia) plant;
c. selecting a mutant *Euphorbia pulcherrima (*Poinsettia*)* plant of step b., wherein at least one allele of the EpGST gene comprises a CTTC deletion in the stretch of 12 nucleotides consisting of a threefold CTTC repeat as present in the genome of the *Euphorbia pulcherrima (*Poinsettia*)* plant of step a.;
d. optionally repeating steps b. and c. until all alleles of the EpGST gene in the plant genome comprise the said CTTC deletion;
e. selecting a *Euphorbia pulcherrima (*Poinsettia*)* plant with white foliage phenotype being homozygous for the EpGST gene comprising the said CTTC deletion, and
f. optionally further asexually propagating said *Euphorbia pulcherrima (*Poinsettia*)* plant being homozygous for the EpGST gene comprising the said CTTC deletion.

As first step, a plant without the white foliage phenotype is provided. This plant may have other desired traits, that should not be lost when generating the white foliage phenotype from such a plant. As it was found that the white foliage trait (i.e. dysfunctional EpGST genes having the CTTC deletion) is recessive, the said plant may be homozygous or heterozygous for the functional EpGST gene. At least one round of mutagenesis is necessary in order to arrive at the plant with white foliage phenotype, performed as step b. In this step, either one or more EpGST alleles are mutated. However, as indicted above, the chance that two alleles are both mutated in a single round of mutagenesis is neglectable.

The invention is based on the surprising finding that despite the use of random mutagenesis, the mutations in the EpGST gene are highly reproducible and consistently resemble a deletion of 4 specific nucleotides (CTTC). The truncation occurs in a part of the GST gene which provides a threefold repeat of these 4 nucleotides (i.e., CTTC-CTTC-CTTC) on positions 128 - 139 of the EpGST cDNA of SEQ ID No 2 and on positions 165 - 178 of the gene sequence of SEQ ID No 1. This deletion causes a frameshift and a functional knock-out of the GST target gene. In fact, the inventors have found that a random method of mutagenesis results in a targeted mutation when applied to the EpGST gene in plants expressing the encoded protein of a functional homolog or variant thereof as defined above, such as the EpGST gene of Poinsettia. The high reproducibility suggests a "hot spot" of mutation i.e., a region of DNA that exhibits an unusually high propensity to mutate. The surprising finding that the knock-out mutation in Poinsettia is highly reproducible provides a method for establishing targeted mutagenesis e.g. by using random mutagenesis techniques, or gene editing techniques therewith providing a highly efficient method to create plants with the white foliage phenotype, in particular Poinsettia plants.

The mutagenesis treatment involves the CTTC deletion by human intervention, which deletion reduces the expression or activity of the EpGST. After mutagenesis step b., a mutant plant is selected in step c., wherein at least one copy of the EpGST gene is dysfunctional as a result of the CTTC deletion, resulting in abolishment of the activity of the encoded GST enzyme. As the gene of interest is known, i.e. an EpGST gene as defined above, the skilled person will be capable of identifying plants wherein such proteins are expressed as well as identifying whether at least one of the said alleles is dysfunctional as a result of the CTTC deletion. Additional rounds of mutagenesis are to be performed, in order to obtain the envisaged mutations in each allele. When the starting plant is homozygous for the functional EpGST gene, two rounds of mutagenesis are usually needed in the diploid Poinsettia. Therefore, step b. and c. are repeated accordingly, followed by a final selection step to verify that the obtained mutant is homozygous for the dysfunctional GST gene, i.e. having the CTTC deletion. In case of polyploid varieties, additional rounds of mutation can be necessary to obtain the envisaged homozygous mutant.

The invention not only identifies the molecular mechanism for the white foliage phenotype, it also provides reproducible methods how to generate such phenotype in any elite plant variety irrespective of the allele combination thereof (i.e., whether the plant is heterozygous or homozygous for the functional allele).

The method allows to convert elite lines into their white coloured derivatives without the need for crossing and backcrossing, thereby preserving all the benefits of the starting material. This is especially beneficial for lines which are vegetatively propagated e.g., chimeras etc. In consequence, the method of the invention allows to establish white coloured derivatives of elite lines which are genetically identical to the starting elite material except for the desired genetic change, and any off-target change caused by method of mutagenesis and any natural change during vegetative propagation.

It is one specific benefit of the invention that an original non-white (e.g., red or pink) plant can be converted into a white-foliar phenotype plant under the retention of all its others phenotypic and morphologic characteristics. Prior to this invention the creation of a white-foliar plant required substantial crossing and selection which always changed the phenotypic and morphologic characteristics of the original plant. In consequence the present invention is especially useful when applied to elite varieties with high performance and/or high consumer acceptance as the invention allows to change solely the foliar colour without touching any other phenotypic and morphologic characteristics.

The elite original variety can be homozygous for the functional GST gene or heterozygous (i.e., comprising already one recessive copy of a dysfunctional GST gene). The *Euphorbia pulcherrima* plant provided in step a. is heterozygous for the EpGST gene, comprising one functional allele as well as one dysfunctional allele comprising the CTTC deletion.

In a preferred embodiment the plant provided in step a. of the method of the invention is derived from an elite Euphorbia variety selected from the group consisting of those presented in the following Table 1:

| | **Trade Name** | **Denomination** | **Breeder** |
|---|---|---|---|
| 1 | Christmas Magic | | Albani |
| 2 | Aries Red | | Beekenkamp |
| 3 | Astro Red | | Beekenkamp |
| 4 | Blissful Red | | Beekenkamp |
| 5 | Charon Red | | Beekenkamp |
| 6 | Hera Red | | Beekenkamp |
| 7 | Leona Red | | Beekenkamp |
| 8 | Robyn Red | | Beekenkamp |
| 9 | Solar Red | | Beekenkamp |
| 10 | Bella Italia | | Dümmen Orange |
| 11 | Bouquet | | Dümmen Orange |
| 12 | Bravo Bright Red | | Dümmen Orange |
| 13 | Burning Ember | | Dümmen Orange |
| 14 | Embla | | Dümmen Orange |
| 15 | EuroGlory Red | | Dümmen Orange |
| 16 | Ferrara | | Dümmen Orange |
| 17 | Freedom Red | | Dümmen Orange |
| 18 | Freya Red | | Dümmen Orange |
| 19 | Grande Italia | | Dümmen Orange |
| 20 | Jubilee Red | | Dümmen Orange |
| 21 | Infinity Red 2.0 | | Dümmen Orange |
| 22 | Maxima Red | | Dümmen Orange |
| 23 | Maximax | | Dümmen Orange |
| 24 | Ouverture Red | | Dümmen Orange |
| 25 | Premium Red | | Dümmen Orange |
| 26 | Prestige Sunrise | | Dümmen Orange |
| 27 | Prima Donna | | Dümmen Orange |
| 28 | Prima Vera | | Dümmen Orange |
| 29 | Scandic Early | | Dümmen Orange |
| 30 | Primero Red | | Dümmen Orange |
| 31 | Viking Red | | Dümmen Orange |
| 32 | Advantage Red | | Dümmen Orange |
| 33 | Advent Red | | Dümmen Orange |
| 34 | Matinée Bright Red | | Dümmen Orange |
| 35 | Prima Bella | | Dümmen Orange |
| 36 | Tikal Red | | Dümmen Orange |
| 37 | Prestige Early | | Dümmen Orange |
| 38 | Winter Rose Early Red | | Dümmen Orange |
| 39 | Red Blitz | | Florensis |
| 40 | Red Joy | | Florensis |
| 41 | Q-ismas Bond | | Graff |
| 42 | Q-ismas Kiss | | Graff |
| 43 | Q-ismas Crunch Red | | Graff |
| 44 | Futura Red | | Lazzeri |
| 45 | Allegra Early Red | | Lazzeri |
| 46 | Superba Red | Lazzpo1066 | Lazzeri |
| 47 | Serena Red | Lazzpo1110 | Lazzeri |
| 48 | Rubino Red | Lazzpo1064 | Lazzeri |
| 49 | Opera Red | Lazzpo1301 | Lazzeri |
| 50 | Roccostar Bright Red | | Lazzeri |
| 51 | Santo | | Psenner |
| 52 | Mito | | Psenner |
| 53 | Gloriette Glamour | | Psenner |
| 54 | Mars Early Red | | Syngenta |
| 55 | Early Millennium | | Syngenta |
| 56 | Lyra Red | | Syngenta |
| 57 | Magma Red | | Syngenta |
| 58 | Marjoris Red | | Syngenta |
| 59 | Mars Red | | Syngenta |
| 60 | Mira Red | | Syngenta |
| 61 | Mirage Red | | Syngenta |
| 62 | Orion Early Red | | Syngenta |
| 63 | Neva Red | | Syngenta |
| 64 | Sigma Red | | Syngenta |
| 65 | Saga Red | | Syngenta |
| 66 | Titan Red | | Syngenta |
| 67 | Vega Red | | Syngenta |
| 68 | Pepeta | | Rinehart Poinsettias |
| 69 | Legacy | | Rinehart Poinsettias |
| 70 | Christmas Day | NPCW10164 | Klemm + Sohn |
| 71 | SK 191 | | Klemm + Sohn |
| 72 | SK 185 | | Klemm + Sohn |
| 73 | Christmas Eve | NPCW08153 | Klemm + Sohn |
| 74 | Noel | NPCW10167 | Klemm + Sohn |
| 75 | Christmas Aurora^{®} | NPCW14221 | Klemm + Sohn |
| 76 | Holy Day /Christmas Wish | NPCW13218 | Klemm + Sohn |
| 77 | Christmas Cracker | NPCW17257 | Klemm + Sohn |
| 78 | Christmas Universe/Christmas Bells | NPCW19282 | Klemm + Sohn |
| 79 | Christmas Angel | NPCW20275 | Klemm + Sohn |
| 80 | Happy Mood | N PC W 20344 | Klemm + Sohn |

Additional elite varieties can be readily identified using the variety finders of the EU Community Plant Variety Office (https://cpvo.europa.eu/en/applications-and-examinations/cpvo-variety-finder), UPOV (https://www.upov.int/pluto/en/), USDA (https://www.ams.usda.gov/datasets/plant-variety), USPTO http://patft.uspto.gov/, or other databases known to the person skilled in the art, electronic catalogues, and internet resources .

In a preferred embodiment, the homolog or variant of the protein encoded by the EpGST gene further has a V on position 2 of SEQ ID No 3, and/or an F or an L on position 62 of SEQ ID No 3, and/or LE on positions 90 - 91 of SEQ ID No 3, and/or an S on position 153 of SEQ ID No 3, as these domains also play a role in the anthocyanin binding of the GST enzyme.

Preferably, the EpGST cDNA has the nucleotide sequence of SEQ ID No 1. In that case, the dysfunctional EpGST gene comprises a CTTC deletion within positions 128-139 of SEQ ID No 1. Accordingly, the EpGST gene preferably has the nucleotide sequence of SEQ ID No 61.

In another attractive embodiment of the invention, step a. further comprises the step of determining whether at least one EpGST gene in the provided plant has the CTTC deletion before being subjected to the mutagenesis treatment of step b., and wherein step c. comprises selecting for a mutant wherein at least two copies of the EpGST gene of step a. have the CTTC deletion. By determining whether at least one EpGST gene is dysfunctional in the provided plant before being subjected to the first round of mutagenesis, it can be established whether the provided plant is homozygous or heterozygous for the functional EpGST gene. If the provided plant is diploid and appears to be heterozygous, only a single round of mutation is necessary to obtain the homozygous dysfunctional GST mutant. In that case, step c. coincides with step e., as in step c. a selection is made for the presence of two dysfunctional GST alleles, which implies homozygosity for the dysfunctional gene.

Attractively, the mutagenesis in step b. is directed to the CTTC deletion of the EpGST gene of the said plant. The mutagenesis treatment e.g. comprises targeted mutagenesis, directed to the CTTC deletion from the EpGST gene of the said plant. By such a mutagenesis, only the envisaged location on the allele is mutated, leaving the remainder of the genome unaffected. This is of particular interest when an elite variety is to be mutated to the white foliage phenotype, while maintaining the advantageous traits of the said variety.

Additional species of plants, for example those analysed herein with anthocyanin-related GST genes may be targeted for mutagenesis using the methods disclosed herein to generate dysfunctional GST. These species may at least include the species for which the GST target sequences are provided herein: *Cyclamen persicum* x *Cyclamen purpurascens, Vitis vinifera, Litchi chinensis,* and *Prunus persica.*

The target plant could be a variety of red wine (*Vitis vinifera*) which is by use of the method of this invention converted into a white wine variety while otherwise retaining all its other essential characteristics. This approach is especially useful as an alternative for producing "Blanc de noirs" wines, sparkling wines or champagnes. Normally a "Blanc de noirs" (literally "white from blacks") wine is a white wine produced entirely from black grapes. This is possible by careful processing of red grapes, as all the anthocyanins are retained in the skin. However, this processing requires hand-picking and careful treatment and is often not perfect resulting in a slight colouring of the wine. These issues can be resolved by the method as described herein as no red anthocyanin pigments would become incorporated in the skin.

Random mutagenesis that would also affect the other parts of the genome is undesired. The skilled person is well aware of targeted mutagenesis techniques, known in the art, such as e.g. site-directed mutagenesis, CRISPR, Zn-finger mediated mutagenesis, TALEN etc. As explained above, random mutagenesis is also to be understood as targeted mutagenesis in case of the presence of a hot spot where mutations occur preferentially, such as in the present case, where the CTTC stretch appear to be such a hot spot. In such a case, radiation mutagenesis or chemical mutagenesis e.g. by ethyl methane sulphonate can be suitable techniques but should preferably be checked using suitable genetic markers. The mutagenesis conditions can be controlled in order to target the mutagenesis. How to control the mutagenesis conditions is known to the skilled person. It has also been shown that mutagenesis using intercalating agents such as acridines, proflavine, ICR191 and ethidium bromide may preferentially occur at hot spots, resulting in a frameshift mutation rendering such mutagenesis technique to be suitable as well (see e.g. http://www.bio.brandeis.edu/classes/biol122a/Lecturerepeats.htm). Other techniques such as gene editing are also possible and lie well within the scope of the skilled person.

Accordingly, the plant of step a. is preferably genetically identical to the plant obtained in step c. and e., apart from the CTTC deletion in the EpGST gene.

The invention also provides a white-foliaged *Euphorbia pulcherrima (*Poinsettia*)* plant derived from a non-white foliaged cultivated *Euphorbia pulcherrima (*Poinsettia*)* plant, said non-white foliaged cultivated *Euphorbia pulcherrima (*Poinsettia*)* plant comprising in its genome a glutathione S-transferase gene EpGST encoding a protein of SEQ ID No 3, or encoding a functional homolog or variant of said protein at least 90%, preferably 95% amino acid identity with the said protein of SEQ ID No 3, the EpGST gene comprising, in the region encoding amino acids at positions 40 - 50 of the protein of SEQ ID No 3, a stretch of 12 nucleotides consisting of a threefold CTTC repeat; the white-foliaged derivative plant being obtainable by the method as described above, which derivative plant is genetically identical to the non-white foliaged cultivated plant, but for
a. In each allele of the said EpGST gene a CTTC deletion in the stretch of 12 nucleotides consisting of a threefold CTTC repeat as present in the genome of the non-white foliaged cultivated plant,
b. any off-target genetic change caused by method of mutagenesis and
c. any natural genetic change during propagation.

In particular, such a white-foliaged *Euphorbia pulcherrima* comprises in its genome a gene encoding a functional homolog or variant of the protein of SEQ ID No 3 with at least 90%, preferably 95% amino acid identity with the said protein of SEQ ID No 3, the said homolog or variant having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, the said gene comprising, in the region encoding amino acids at positions 40 - 50 of the protein of SEQ ID No 3, a stretch of 12 nucleotides consisting of a threefold CTTC repeat.

In another embodiment, such a white-foliaged *Euphorbia pulcherrima* comprises in each allele of the said EpGST gene a CTTC deletion in the stretch of 12 nucleotides consisting of a threefold CTTC repeat as present in the genome of the non-white foliaged cultivated *Euphorbia pulcherrima (*Poinsettia*)* plant, and wherein said white-foliaged *Euphorbia pulcherrima (*Poinsettia*)* plant has otherwise all of the phenotypic and morphologic characteristics of the non-white foliaged *Euphorbia pulcherrima (*Poinsettia*)* plant.

In another embodiment, the said white-foliaged plant as described above has, otherwise all of the phenotypic and morphologic characteristics of the non-white foliaged plant.

Another embodiment provides *Euphorbia pulcherrima* with a white foliage phenotype, derived from a plant with white foliage phenotype as described above as first donor plant by breeding technologies with one or more non-white foliaged second donor *Euphorbia pulcherrima (*Poinsettia*)* plants comprising one or more elite properties, wherein said derived plant comprises in each allele of the said EpGST gene a CTTC deletion, wherein the derived plant has one or more elite properties from the one or more second donor plants.

The invention also relates to a cultivated plant having a white foliage phenotype, being homozygous for the EpGST gene having the CTTC deletion, obtainable by the method of the above-described method where the wild type gene in the said plant has the threefold CTTC repeat.

The invention also relates to seeds, plant parts, plant cells or a plant population derived from a plant as described above. Plants parts can be any part of the plant, such as fruits, stems, foliage, roots etcetera. Chemically isolated constituents are not to be regarded as such plant parts.

In a preferred embodiment, the CTTC deletion causing the white foliage phenotype of the *Euphorbia pulcherrima (*Poinsettia*)* plant of the invention is not the exclusively the result of a sexual crossing of whole genomes, but is obtained by a method of mutagenesis as described above, preferably by a method of man-induced mutagenesis. In a further preferred embodiment the plant of the invention is not exclusively produced by means of an essentially biological process. The term "exclusively" in this context means that the method of establishing the white foliage phenotype and or the plant of the invention is not entirely based on the crossing of whole genomes (i.e., an essentially biological process), but that the process contains - preferably within the steps of sexually crossing and selecting - an additional step of a technical nature, which step by itself introduces a trait into the genome or modifies a trait in the genome of the plant produced, so that the introduction or modification of that trait is not the result of the mixing of the genes of the plants chosen for sexual crossing. More preferably the step of technical nature is the mutagenesis step b.

In another embodiment, the invention also provides a molecular marker capable of identifying an EpGST gene comprising the CTTC deletion as defined above. In particular, the marker is capable of identifying the CTTC deletion within positions 128-139 of the EpGST gene of SEQ ID N0 1, or the cDNA of SEQ ID No2. Such a marker can advantageously be used for the determination of the homo- or heterozygosity of the envisaged GST alleles.

The above marker is attractively used in the selection step c. of the method as described above.

In another embodiment, the invention provides isolated DNA of the EpGST gene comprising a CTTC deletion as identified above. In particular, the isolated DNA comprises the CTTC deletion within positions 128-139 of SEQ ID No 1, and the use of such DNA for the preparation of a molecular marker capable of identifying the dysfunctional EpGST gene comprising a CTTC deletion within positions 128-139 of SEQ ID No 1.

A further embodiment of the invention relates to the genomic GST gene of *Euphorbia pulcherrima* GST (*EpGST*)*.* Preferred is the isolated genomic DNA of the EpGST gene described by SEQ ID NO: 61 or a variant thereof having at least 90%, preferably 95% amino acid identity to the sequence described by SEQ ID NO: 61, or a fragment of at least 20 continuous nucleotides thereof. Further preferred is the isolated DNA of the EpGST gene comprising a CTTC deletion, more preferable where the CTTC deletion within positions 128-139 of SEQ ID No 1.

Herein, the term "EpGST" relates to the glutathione S-transferase of Euphorbia pulcherrima, whereas the term 'GST' relates to a glutathione S-transferase having a first domain defined at positions 11 - 13 of SEQ ID No 3 being AAC, AGC or AAN, where N can be any amino acid, a second domain defined at positions 53 - 56 of SEQ ID No 3 being LVPA, QVPA or QPVP and a third amino acid domain defined at positions 65 - 68 of SEQ ID No 3 being FESR and having glutathione transferase activity as defined above.

A further embodiment of the invention relates to the cDNA sequence of the *Euphorbia pulcherrima* GST gene (*EpGST*)*.* Preferred is the isolated cDNA encoding a EpGST protein described by SEQ ID NO: 3 or a functional homolog or variant of said protein with at least 90% amino acid identity, the said homolog or variant having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, or fragment of at least 20 continuous nucleotides of said cDNA.

The isolated DNA sequences of the invention and their fragments can be used for multiple purposes including but not limited to design markers, probes, guide RNAs and other tools for the detection and/or modification of the EpGST gene. In a preferred embodiment the isolated DNA sequences are used to design molecular tools for targeted mutagenesis of the EpGST gene, most preferably to deactivate said gene to achieve a white or essentially white phenotype. In a preferred embodiment wherein the mutation in an EpGST gene is selected from the group consisting of a loss-of-function mutation, a partial loss-of-function mutation, a restored frameshift mutation, and an in-frame deletion mutation.

The invention therefore relates to a method of use of the isolated DNA as described above as well as the sequences of SEQ ID NO: 44 to 49, or variants with at least 95% identity therewith for the preparation of a molecular marker as described above or for a method for targeted mutagenesis of a GST gene in a target plant, preferably using a continuous stretch of at least 17 nucleotides from any said isolated DNA sequences to (a) produce a guide RNA or an expression construct therefor for a CRISPR/Cas-based method of gene editing or (b) produce a silencing RNA or an expression construct therefor for a method of RNA-mediated gene silencing.

Also described is a method of for the production of plants having a reduced level of anthocyanins comprising the steps of:
a. providing a plant comprising in its genome at least one functional copy of a glutathione S-transferase GST gene encoding a protein selected from the group consisting of SEQ ID No 3, and 53 to 59 or encoding a functional homolog or variant of said protein with at least 90%, preferably 95% amino acid identity, the said homolog or variant having a first domain corresponding to positions 11 - 13 of SEQ ID No 3 being AAC, AGC or AAN, where N can be any amino acid, a second domain corresponding to positions 53 - 56 of SEQ ID No 3 being LVPA, QVPA or QPVP and a third amino acid domain corresponding to positions 65 - 68 of SEQ ID No 3 being FESR;
b. subjecting the plant of step a. to targeted mutagenesis treatment to produce a mutant GST gene therein, and
c. selecting a plant with reduced level of anthocyanins being homozygous for mutated EpGST gene.

By the finding that plants, being homozygous for the GST gene corresponding to that of the EpGST gene of *Euphorbia pulcherrima* (based on the presence of the above-mentioned domains and functionality) will have a phenotype wherein the anthocyanin mediated colour will be significantly decreased or abolished, such as e.g. the case for *Vitis vinifera,* where the functional loss of the said GST results in white grapes of varieties that are red in case the corresponding functional GST gene would be present.

The mutation in the GST gene is preferably selected from the group consisting of a loss-of-function mutation, a partial loss-of-function mutation, a restored frameshift mutation, an in-frame deletion mutation, or a promoter deletion.

The mutagenesis preferably involves the use of at least one DNA sequence selected from the group consisting of:
(i) the sequences of any of the claims 11 - 13,
(ii) the sequences of SEQ ID NO: 44 to 49, or variants thereof with at least 95% identity,
(iii) the complements to the sequences under (i) and (ii), and
(iv) a fragment of the sequences under (i) to (iii) of at least 17 contiguous nucleotides,
wherein said DNA sequence is preferably used to produce a guide RNA targeting said EpGST.

Mutagenesis may be performed in accordance with any of the techniques known in the art, such as, and not limited to, synthesizing an oligonucleotide having one or more mutations within the sequence of a particular regulatory sequence. In particular, site-specific mutagenesis is a technique useful in the preparation of promoter mutants, through specific mutagenesis of the underlying DNA. RNA-guided endonucleases ("RGEN," e.g., CRISPR/Cas9) may also be used. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered.

In one aspect, the mutagenesis is introduced by a site-directed nuclease. The site-directed nuclease is preferably CRISPR-based, but could also be a meganuclease, a transcription-activator like effector nuclease (TALEN), or a zinc finger nuclease. The nuclease used in this invention could be Cas9, Cfp1, dCas9-Fokl, chimeric FEN1-Fokl. In one aspect, the DNA modification enzyme is a site-directed base editing enzyme such as Cas9-cytidine deaminase or Cas9-adeninie deaminase, wherein the Cas9 can have one or both of its nuclease activity inactivated, i.e. chimeric Cas9 nickase (nCas9) or deactivated Cas9 (dCas9) fused to cytidine deaminase or adenine deaminase. The optional guide RNA targets the genome at the specific site intended to be edited. In one aspect, the optional guide RNA comprises an 18-21 nucleotide sequence with homology to any of SEQ ID NOs: 1, 2 and 61 or the complement thereof.

### Short description of the SEQ IDs

SEQ ID Nos 1 and 2 are the wild type EpGST gene and the corresponding cDNA of Poinsettia, respectively.
SEQ ID No 3 is the amino acid sequence of the EpGST encoded protein
SEQ ID Nos 4-12 and 60 are amplification primers,
SEQ ID Nos 13 - 18 are functional EpGST cDNAs of coloured Poinsettia varieties,
SEQ ID Nos 19 - 24 and 41 are mutant EpGST cDNAs of white Poinsettia varieties,
SEQ ID Nos 25 - 41 are mutant EpGST cDNAs of white and coloured Princettia varieties,
SEQ ID Nos 42 - 49 depict related GST cDNA from plants of other species,
SEQ ID No 50 relates to the amino acid sequence of a C-truncated mutant dysfunctional protein as a result of the CTTC deletion on positions 136 - 139 of the CDNA of SEQ ID No 2, resulting in a frame shifted stop codon at positions 158 - 160.
SEQ ID No 51 relates to the amino acid sequence of an N-truncated mutant dysfunctional protein as a result of the CTTC deletion on positions 136-139 of the CDNA of SEQ ID No 2, resulting in a new in-frame start codon at positions 211 - 213.
SEQ ID NO 52-59 are encoded proteins of the cDNAs of SEQ ID Nos 42 - 49, respectively.
SEQ ID NO 61 is the genomic DNA of the wild type EpGST gene including a 5'UTR stretch of 37 nucleotides.

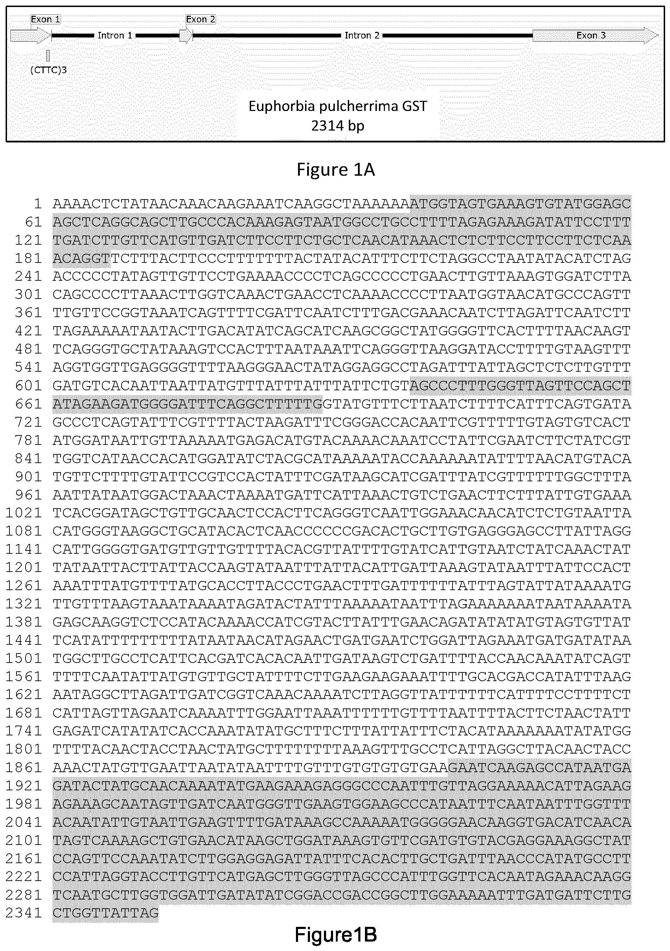

The invention will now be further explained by way of the following figures and examples, wherein
Figure 1A is a schematic representation of the full-length sequence (2314 bp) of the *Euphorbia pulcherrima* GST gene (*EpGST*)*.* Arrows represent the exon regions. Black lines represent the intron regions. The lower square represents the location of the trinucleotide motif SSR locus (CTTC₃). Figure 1B shows the corresponding genomic gene sequence as depicted in SEQ ID No 61 wherein the exons are in grey. Exon 1: nt 38 - 186, exon 2: nt638 - 688, exon 3: nt 1902 - 2351.

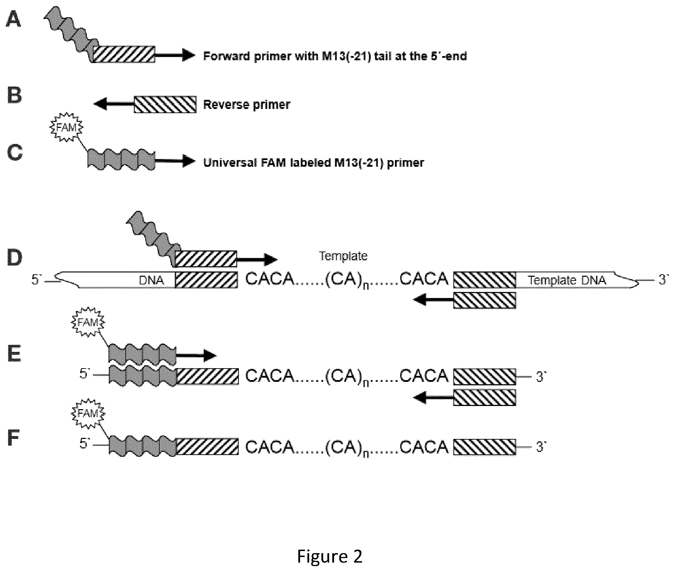

Figure 2 is an amplification scheme for the fluorescent labelling of PCR fragments. A), B) The hatched boxes indicate the *indel*-specific primers. C) The undulating grey box indicates the universal M13(-21) sequence, and the star the fluorescent FAM label. D) In the first PCR cycles, the forward primer with the M13(-21) tail is incorporated into the PCR products. E) These products are then the target for the FAM-labelled universal M13(-21) primer, which is incorporated during subsequent cycles at a lower annealing temperature of 53°C. F) The final labelled product can be analysed on a laser detection system (Figure adapted from Schuelke, (2000), Nature Biotechnol 18:233-234).

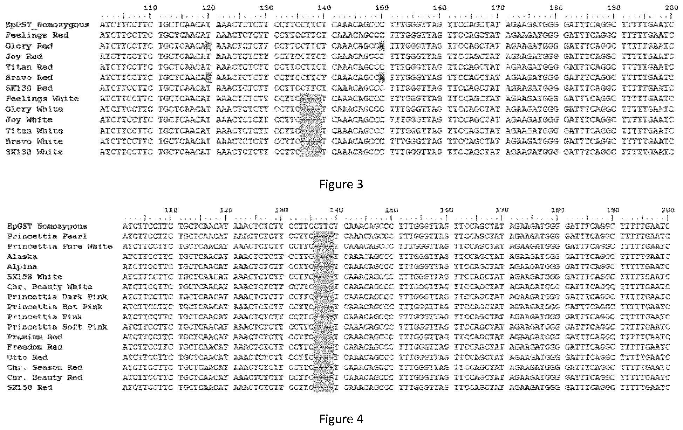

Figure 3 represents an alignment of the *EpGST* CDS for 12 red- and white-bracted Poinsettia genotypes. The figure shows a 100 bp region of the CDS where a 4 bp *indel* is observed only in the white genotypes. The first sequence (named RNASeq_Homozygous) corresponds to the *EpGST* from the homozygous variety Vintage (Klemm + Sohn) and was used as a reference for the alignment.
Figure 4 represents an alignment of the *EpGST* CDS for 16 pink-, red- and white-bracted Poinsettia/Princettia genotypes. The figure shows a 100 bp region of the CDS where a 4 bp *indel* is observed in all sequenced genotypes. The first sequence (named Homozygous) corresponds to the *EpGST* from the homozygous Vintage genotype and was used as a reference for the alignment.

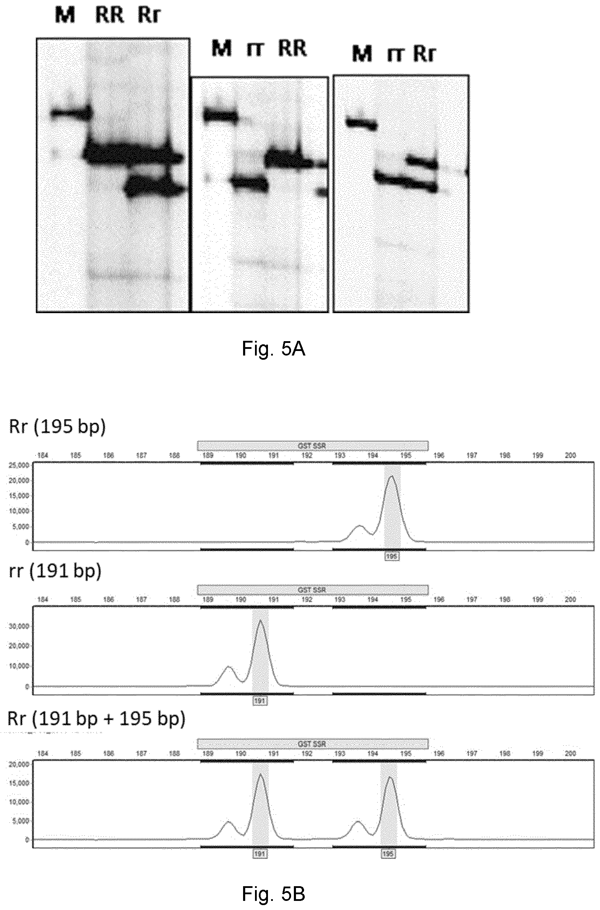

Figure 5 shows exemplary results of a PCR amplification of the trinucleotide motif SSR locus (CTTC₃) in *EpGST* for homozygous red (RR), heterozygous red (Rr), and homozygous white (rr) Poinsettia genotypes. Figure 5A shows PCR amplicons resolved in 6% (w/v) acrylamide gel in vertical electrophoresis; and figure 5B shows PCR amplicons resolved by capillary electrophoresis. M = marker; rr = recessive homozygous genotype for the SSR locus in the *EpGST*; Rr = heterozygous genotype for the SSR locus in the *EpGST*; RR = dominant homozygous genotype for the SSR locus in the *EpGST.*

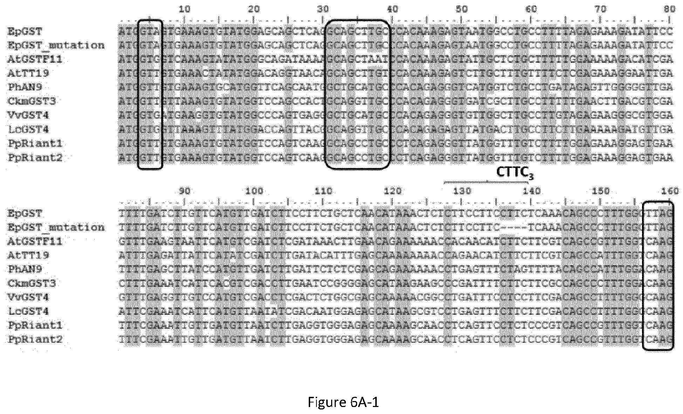

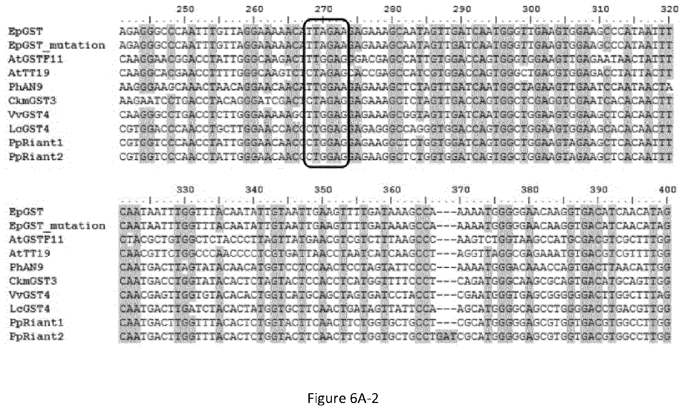

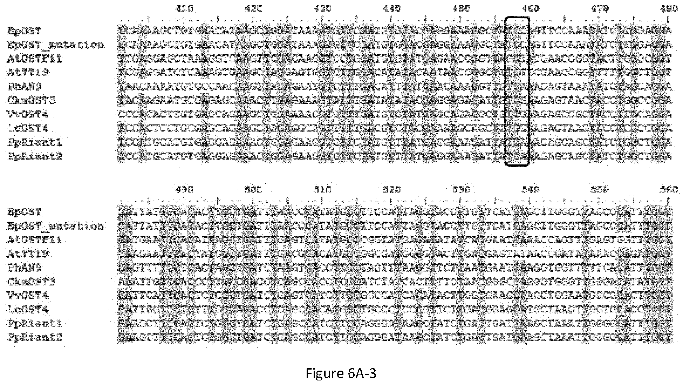

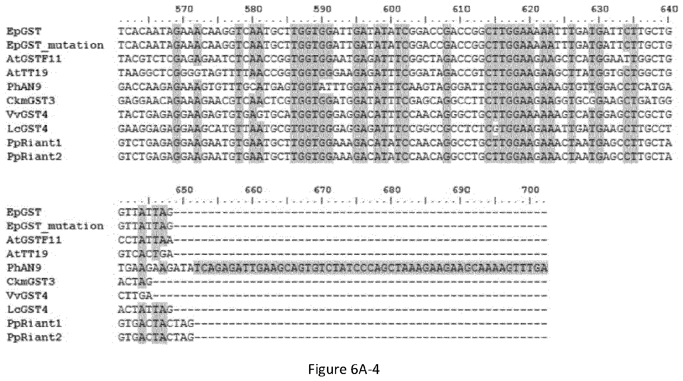

Figures 6A-1 to A-4 and 6B represent a sequence alignment and phylogenetic tree of nucleotide sequences from the *EpGST* gene. A) CLUSTALW sequence alignment of the *EpGST* nucleotide sequence, both wild type and mutated (first and second row, respectively) with known anthocyanin-related GST genes from other plant species: AtGSTF11, AtTT19, PhAN9, CkmGST3, VvGST4, LcGST4, PpRiant1 and PpRiant2 (rows 3 - 10, respectively). The CTTC₃ SSR locus is marked above the alignment. Conserved sequences among all the genes are shown with a grey background. Sequences encoding anthocyanin binding domains are indicated in boxes. B) Constructed Neighbour-Joining tree of the *EpGST* CDS nucleotide sequence with known anthocyanin-related GST genes from other plant species. Bootstrap values were calculated from 1000 replicate analyses and are shown under the tree branches.

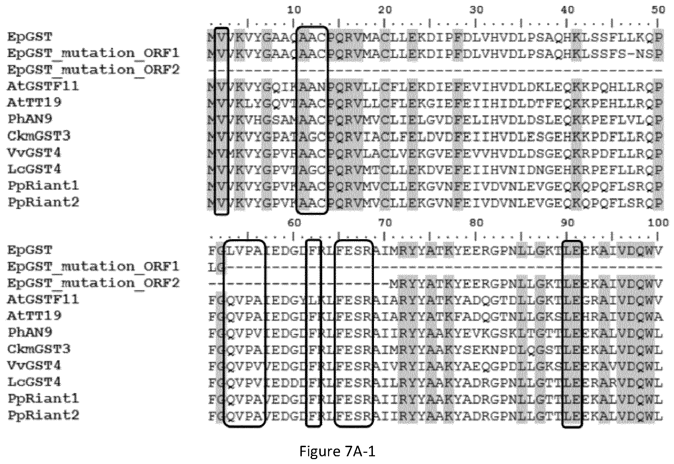

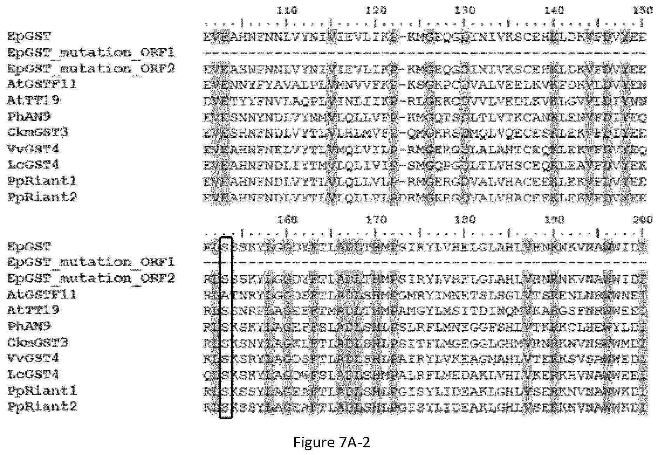

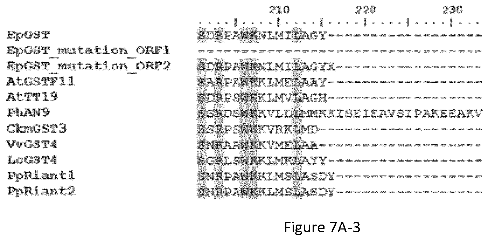

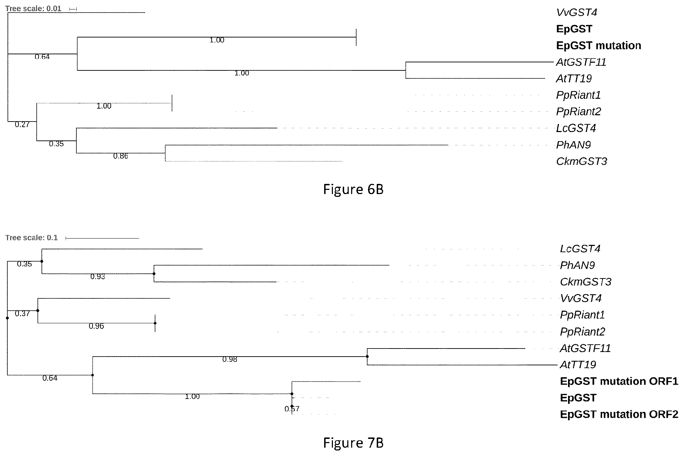

Figures 7A-1 to A-3 and 7B-represent a sequence alignment and phylogenetic tree of deducted amino acid sequences from the *EpGST* and known anthocyanin-related GST genes. A) CLUSTALW sequence alignment of the *EpGST* amino acid sequence with the known anthocyanin-related genes as depicted in figure 6. Conserved sequences among all the genes are shown with a grey background. Anthocyanin binding domains are indicated in boxes B) Constructed Neighbour-Joining tree of the *EpGST* amino acid sequence with known anthocyanin-related genes. Bootstrap values were calculated from 1000 replicate analyses and are shown under the tree branches.

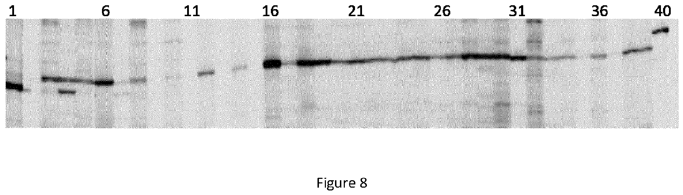

Figure 8 shows the result of SSR marker genotyping of 35 plants recovered after irradiation of the homozygous red variety Christmas Aurora (Klemm + Sohn), randomly selected out of 200 recovered plants. Lanes 1 - 5: controls (lane 1: white rr plant; lane 2: negative control; lane 3: red RR plant; lane 4: red Rr plant; lane 5: original variety Christmas Aurora); lanes 6 - 40: recovered candidate plants.

### Examples

### Example 1: Determination gene and amino acid sequence of EpGST from Poinsettia

To characterize the full-length sequence of the anthocyanin-related GST gene (*EpGST,* SEQ ID No 1), DNA from the red-bracted variety Vintage (Klemm + Sohn) was isolated from approximately 100 mg of leaf tissue using the NucleoSpin^{®} Plant II kit (Macherey-Nagel GmbH & Co. KG, Germany) according to the manufacturer's instructions. The DNA concentration was analysed using NanoDrop^{™} 2000 (Thermo Fisher Scientific, USA) and gel electrophoresis.

Primers were designed from available DNA sequences previously obtained at the Institute for Plant Genetics of the Leibniz University of Hannover (Germany). The sequences of the primers used to amplify the full-length gene are F1 (TCCGATCTAAGAAATCAAGGCTA - forward, SEQ ID No 4) and R1 (CAGTCGGCCGCTACATAGAT - reverse, SEQ ID No 5). Two other primer pairs flanking the intronic regions were used in order to amplify smaller inner fragments to assure the correct sequencing: F2 (TGGCCTGCCTTTTAGAGAAA, SEQ ID No 6) and R2 (AAAGCCTGAAATCCCCATCT, SEQ ID No 7); F2 and R3 (TATGGGCTTCCACTTCAACC, SEQ ID No 8). The PCR reactions were performed in a 50 µL reaction containing 50 ng of DNA template, 1X PrimeSTAR^{®} Buffer (Mg²⁺ plus), 0.2 mM of each dNTP, 0.25 µM of forward and reverse primers and 1.25 U of PrimeSTAR^{®} HS DNA Polymerase. The cycling conditions were 95°C for 3 min; 30 cycles of 95°C for 30 sec, 60°C for 30 sec and 72°C for 2 min; and a final extension of 10 min at 72°C. The PCR products were resolved in a 1% (w/v) agarose gel in horizontal electrophoresis for 90 min at 100 V. The correct bands were excised from the gel and purified using the NucleoSpin^{®} Gel and PCR Clean-up (Macherey-Nagel GmbH & Co. KG, Germany) following manufacturer's recommendations. Finally, the purified PCR fragments were sent to Eurofins Genomics (Ebersberg, Germany) for Sanger sequencing. The generated sequences were aligned using BioEdit Sequence Alignment Editor v7.2.5 and a final full-length gene sequence for the *EpGST* was generated.

The *EpGST* full-length sequence contains three exons (147 bp, 48 bp and 450 bp, respectively) and two introns (455 bp and 1214 bp, respectively) (Figure 1). The whole CDS has a size of 645 bp (SEQ ID No 2), which encodes for a putative protein of 214 amino acids (SEQ ID No 3) and mass of 24.6 kDa.

### Example 2: Detection of a Single Sequence Repeat locus in the EpGST sequence

The colour range in Poinsettia varieties is obtained either through classical breeding (crossing) or mutagenic breeding (radiation), thus generating a spectrum of bract colours, such as pink, marble, orange and white/creamy. The white genotypes are often obtained through several rounds of radiation mutagenesis starting with a plant having the red genotype, followed by shoot development and trait selection. Therefore, red and white Poinsettias from the same genotype are referred to as 'pairs', due to their highly similar genetic background.

6 Pairs of red- and white-bracted varieties of Poinsettia (Christmas Feelings (SEQ ID No 13), Christmas Feelings White (SEQ ID No 19), Christmas Glory (SEQ ID No 14), Christmas Glory White (SEQ ID No 20), Christmas Joy (SEQ ID No 15), Christmas Joy White (SEQ ID No 21), SK130 (SEQ ID No 18) and SK130 White (SEQ ID No 24), all from Klemm + Sohn; Titan Red (SEQ ID No 16) and Titan White (SEQ ID No 22) from Syngenta; and Bravo Bright Red (SEQ ID No 17) and Bravo White (SEQ ID No 23) from Dümmen Orange) were used to analyse the coding sequence (CDS) of the *EpGST* gene (SEQ ID No 2). Total RNA was isolated from approximately 100 mg of bract tissue using the mirPremier^{™} miRNA isolation kit (Sigma-Aldrich, USA) according to the manufacturer's instructions. The total RNA concentration was analysed using NanoDrop^{™} 2000 (Thermo Fisher Scientific, USA). cDNA synthesis was performed using the FastGene Scriptase Basic cDNA Kit (Nippon Genetics Europe GmbH, Germany) according to the manufacturer's recommendations. The primers used for the PCR amplification were F1 (TCCGATCTAAGAAATCAAGGCTA, SEQ ID No 4) and R1 (CAGTCGGCCGCTACATAGAT, SEQ ID No 5). PCR amplification protocol and sequencing strategies were the same used for the previous analysis.

The sequence alignment of the CDS of the EpGST showed high similarity for all genotypes. However, a 4 bp deletion located 8 bp upstream the first exon-intron junction was observed in all white genotypes (Figure 3). The deletion is located in a Single Sequence Repeat (SSR) locus, with a trinucleotide motif (CTTC₃) composition. The exact location of the CTTC₃ motif is shown in figure 1. The 4 bp deletion in the SSR locus would result in a putative early stop codon on the amino acid sequence of the GST protein, thus leading to a possible non-functional protein.

Not all red Poinsettia genotypes are able to produce white sports through radiation. Therefore, red Poinsettia genotypes are distinguished into 'heterozygous' and 'homozygous' for the colouration locus according to their ability to generate white sports (heterozygous produce pink to white sports and homozygous do not).

The relation of the 4 bp deletion with the bract colouration in Princettia was investigated by sequencing, the *EpGST* CDS from the following 16 Poinsettia/Princettia genotypes: 1) white genotypes - Princettia Pearl (SEQ ID No 25) and Princettia Pure White (SEQ ID No 26) from Sakata (Japan), Alaska (SEQ ID No 27) and Alpina (SEQ ID No 28) from Lazzeri (Italy), SK158 White (SEQ ID No 29) and Christmas Beauty White (SEQ ID No 30) from Klemm + Sohn (Germany); 2) pink genotypes - Princettia Dark Pink (SEQ ID No 31), Princettia Hot Pink (SEQ ID No 32), Princettia Pink (SEQ ID No 33) and Princettia Soft Pink (SEQ ID No 34) from Sakata (Japan); and 3) red genotypes: Premium (SEQ ID No 35) and Freedom (SEQ ID No 36) from Dümmen Orange (Netherlands), Otto (SEQ ID No 37) from Süptitz (Germany), Christmas Season (SEQ ID No 38), Christmas Beauty (SEQ ID No 39) and SK158 (SEQ ID No 25) from Klemm + Sohn (Germany).

mRNA isolation, cDNA synthesis, PCR amplification and sequencing were the same used for the previous analysis.

All the genotypes showed the 4 bp deletion in the same position (Figure 4). Therefore, a heterozygous locus was believed to be present in the red and pink genotypes.

It was however observed that both Poinsettia varieties Alaska and Alpina, while being white, appeared to be heterozygous for the EpGST gene, i.e. both varieties still having a functional EpGST gene. As indicated above, it is believed that these varieties must have been mutated elsewhere in the genome, e.g. affecting pigment production. The same is true for the white Princettia varieties Pearl and Pure white (RHS colour code of about 155C and about 155C, respectively), indicating that for Princettia, another mode of action applies for the white foliage phenotype.

### Example 3: Genotyping of Poinsettia varieties for SSR locus in the EpGST sequence

To detect the 4 bp deletion in a Single Sequence Repeat (SSR) locus in the *EpGST* sequence which appears to be related to the bract colouration in poinsettia, we applied a genotyping approach based on the fluorescent labelling of PCR fragments (Schuelke, 2000, Nature Biotechnol 18:233-234). The principle of the approach is shown in figure 2.

DNA samples from 78 different commercially available Poinsettia genotypes were isolated from approximately 100 mg of leaf tissue using the NucleoSpin^{®} Plant II kit (Macherey-Nagel GmbH & Co. KG, Germany) according to the manufacturer's instructions. The DNA concentration was analysed using NanoDrop^{™} 2000 (Thermo Fisher Scientific, USA).

Primers were designed surrounding the SSR locus in the *EpGST* sequence, with an M13-tail added at the 5'-end of the forward primer. The sequences for the primers are the following:
F (GTAAAACGACGGCCAGTTGGCCTGCCTTTTAGAGAAA, SEQ ID No 9) and
R (ACAAGTTCAGGGGGCTGAG, SEQ ID No 10).

The PCR reactions were performed in a 20 µl reaction containing 50 ng of DNA template, 1X Williams buffer, 0.15 mM of each dNTP, 0.0125 µM of forward, 0.07 µM of universal FAM labelled M13 primer, 0.25 µM of reverse primers and 1 U of DCSPol DNA Polymerase (DNA Cloning Service, Germany). The cycling conditions were 94°C for 3 min; 24 cycles of 94°C for 45 sec, 59°C for 1 min and 72°C for 1 min; 6 cycles of 94°C for 30 sec, 52°C for 45 sec and 72°C for 1 min; and a final extension of 10 min at 72°C. 50 µl of formamide loading dye was added to each reaction and incubated at 95°C for 5 min. The PCR products were resolved in 6% (w/v) acrylamide gel in vertical electrophoresis using the LI-COR Gene Reader 4200 DNA Analyzer (LI-COR Biosciences, USA).

The electrophoresis analysis results in the generation of a band of 195 bp size in case of the unmutated EpGST allele, whereas the mutated EpGST allele results in a 191 bp DNA fragment, which could clearly be separated from the unmutated EpGST allele by the analysis method (Figure 5A). Here, RR denominates the homozygous presence of only original alleles (band at 195 bp only), Rr the heterozygous presence of the original and the mutated allele (both bands), and rr the homozygous presence of only the mutated allele (band at 191 bp only). M denominates DNA size marker.

The list of all tested genotypes, their elucidated zygosity status for the SSR locus and their respective bract colouration is given in table 2.

**Table 2 Poinsettia varieties tested**

| **ID** | **Genotype name** | **Denomination** | **Zygosity** | **Bract colour** | **RHS colour code** |
|---|---|---|---|---|---|
| 1 | Chr. Feelings Pearl | NPCW13211 | rr | white | 4D |
| 2 | Chr. Glory White | NPCW17267 | rr | white | 8D |
| 3 | Chr. Joy White | | rr | white | |
| 4 | Bravo White | | rr | white | |
| 5 | Titan White | | rr | white | |
| 6 | SK158 White | | rr | white | |
| 7 | SK130 White | | rr | white | |
| 8 | Candlelight | NPCW12202 | rr | white | Between 158B-4D |
| 9 | Whitestar | Fispue White | rr | white - light cream | 4D |
| 10 | Premium Polar | Duepremimpol | rr | white - light cream | Close to NN1155A |
| 11 | Chr. Carol White | | rr | white | |
| 12 | Chr. Feelings White | NPCW08119 | rr | white | 1D |
| 13 | Chr. Star Marble | | rr | white | |
| 14 | SK 136 White | | rr | white | |
| 15 | Vintage | | RR | red | |
| 16 | Chr. Tradition | NPCW14205 | RR | red | |
| 17 | Chr. Aurora | NPCW14221 | RR | red | |
| 18 | Chr. Morning | NPCW15237 | RR | red | |
| 19 | Holy Day | NPCW13218 | RR | red | |
| 20 | Chr. Wish Pink | NPCW18281 | RR | pink | |
| 21 | Grande Italia | | RR | red | |
| 22 | Prima Donna | | RR | red | |
| 23 | Scandic Early | | RR | red | |
| 24 | Early Millennium | | RR | red | |
| 25 | Aries Red | | RR | red | |
| 26 | Blissful Red | | RR | red | |
| 27 | Maxima | | RR | red | |
| 28 | Bouquet | | RR | red | |
| 29 | Ferrara | | RR | red | |
| 30 | Lyra Red | | RR | red | |
| 31 | Tabaluga | | RR | red | |
| 32 | Prestige Red | | RR | red | |
| 33 | Chr. Spirit | NPCW04095 | RR | red | |
| 34 | Chr. Day | NPCW10164 | RR | red | |
| 35 | Chr. Eve | NPCW08153 | RR | red | |
| 36 | Noel | NPCW10167 | RR | red | |
| 37 | Astro Red | | RR | red | |
| 38 | Leona Red | | RR | red | |
| 39 | Viking Red | | RR | red | |
| 40 | Bella Italia | | RR | red | |
| 41 | Burning Ember | | RR | red | |
| 42 | Mirage Red | | RR | red | |
| 43 | Vega Red | | RR | red | |
| 44 | Magma | | RR | red | |
| 45 | Advantage Red | | RR | red | |
| 46 | Chr. Cracker | NPCW17257 | RR | red | |
| 47 | Chr. Magic | NPCW18268 | RR | red | |
| 48 | Chr. Universe | | RR | red | |
| 49 | SK167 | | RR | red | |
| 50 | Chr. Feelings | NPCW02044 | Rr | red | |
| 51 | Chr. Glory | NPCW12200 | Rr | red | |
| 52 | Chr. Joy | NPCW12197 | Rr | red | |
| 53 | Bravo Bright Red | | Rr | red | |
| 54 | Titan Red | | Rr | red | |
| 55 | SK130 | | Rr | red | |
| 56 | SK158 | | Rr | red | |
| 57 | Chr. Carol | NPCW04107 | Rr | red | |
| 58 | Freedom | | Rr | red | |
| 59 | Otto | | Rr | red | |
| 60 | Chr. Season | KLEW01066 | Rr | red | |
| 61 | Mars | | Rr | red | |
| 62 | Mira | | Rr | red | |
| 63 | Chr. Feelings Merlot | | Rr | red | |
| 64 | Chr. Sensation | NPCW18087 | Rr | red | |
| 65 | Chr. Break | | Rr | red | |
| 66 | SK149 | | Rr | red | |
| 67 | Chr. Mouse | | Rr | red | |
| 68 | Chr. Feelings Select | NPCW08122 | Rr | red | |
| 69 | Premium Red | | Rr | red | |
| 70 | Infinity Red 2.0 | | RR | red | |
| 71 | Valentino | NPCW11201 | RR | red | |
| 72 | Scarlet Red | | RR | red | |
| 73 | SK168 | | RR | red | |
| 74 | SK176 | | Rr | red | |
| 75 | SK175 | | Rr | red | |
| 76 | SK172 | | RR | orange | |
| 77 | SK173 | | RR | orange | |
| 78 | SK174 | | RR | orange | |

### Example 4: Phylogenetic analysis of GST genes

Different phylogenetic analyses were performed to evaluate the similarity of the anthocyanin-related *EpGST* with genes encoding GST from different plant species. The following anthocyanin-related GSTs from other species were included in the analysis: *CkmGST3* (*Cyclamen persicum* x *Cyclamen purpurascens* - AB682678.1; SEQ ID No 45), *LcGST4* (*Litchi chinensis* - KT946768.1; SEQ ID No 47), *VvGST4* (*Vitis vinifera -* AY971515.1; SEQ ID No 46), *PhAN9* (*Petunia hybrida* - Y07721.1; SEQ ID No 44), *PpRiant1* (*Prunus persica* - KT312847.1; SEQ ID No 48), *PpRiant2* (*Prunus persica* - KT312848.1; SEQ ID No 49), *AtGSTF11* (*Arabidopsis thaliana* - NM_111189.3; SEQ ID No 42) and *AtTT19* (*Arabidopsis thaliana* - NM_121728.4; SEQ ID No 43). The nucleotide coding sequence of *EpGST* (SEQ ID No 2) and its version containing a 4bp deletion (SEQ ID No 41) as well as the deducted amino acid sequences were compared with the aforementioned GSTs and deducted amino acid sequences from other species (SEQ ID Nos 3 and 50 - 59, respectively).

Sequence alignment was performed using CLUSTAL W and the best DNA/Protein Model was calculated with MEGA v7.0 with the following parameters: i) Tree to use - Neighbour-joining tree and ii) Statistical method - Maximum Likelihood (ML). An ML tree was generated using MEGA v7.0 with bootstrap values calculated from 1000 replicate analyses.

In order to analyse similarities among *EpGST* and anthocyanin-related GSTs from other species (*CkmGST3, LcGST4, VvGST4, PhAN9, PpRiant1, PpRiant2, AtGSTF11* and *AtTT19*) the gene sequences were analysed, and a phylogenetic tree was made, see figures 6 and 7.

GST genes play an important role in anthocyanin transportation, since GST mutants show phenotypes with a visible lack of pigmentation, such as *bz2* (*Bronze-2*) from maize, *an9* (*Anthocyanin 9*) from Petunia, *tt19* (*Transparent Testa 19*) from Arabidopsis and *fl3* (*Flavonoid3*) from Dianthus (Marrs et al., (1995) Nature 375:397-400; Alfenito et al., (1998) Plant Cell 10: 1135-1149; Larsen et al., (2003) Plant Cell Rep 21:900-904; Kitamura et al., (2004) Plant J 37:104-114). Moreover, there is a high functional conservation of GSTs involved in flavonoid accumulation (Zhao (2015) Trends Plant Sci 20(9):576-585). By aligning the CDS nucleotide sequences of both *EpGST* alleles (with and without the 4 bp deletion at the CTTC₃ SSR locus) with anthocyanin-related GSTs from other species, an overall nucleotide similarity of 64% was observed. Moreover, none of the anthocyanin-related genes contains the same CTTC₃ motif as observed in the *EpGST* (Figure 6A). Lastly, the phylogenetic tree shows that the *EpGST* presents more similarity with *AtGSTF11* and *AtTT19* from *A*. *thaliana* than with the other anthocyanin-related genes (Figure 6B).

A further analysis was made with regard to the similarity of the deducted amino acid sequence from the *EpGST* with the amino acid sequences of the same anthocyanin-related genes. The amino acid alignment showed an overall similarity of 61% (Figure 7A). In addition, the phylogenetic tree shows that the deducted amino acid sequence from *EpGST* presents more similarity with *AtGSTF11* and *AtTT19* from *A*. *thaliana* than with the other anthocyanin-related GST genes (Figure 7B).

### Example 5: Generation of white-bracted Poinsettia plants from elite plant material through irradiation

Commercial Poinsettia elite lines, selected for superior characteristics, e.g. high cuttings yield, reliable and fast rooting, very good branching, early flowering and excellent shelf life were chosen as starting material for irradiation treatments to achieve white foliage types maintaining all the characteristics of the elite line. Usually the bract colour of the starting elite line is red but may also be pink or marble or alternatively it may show variations or shades of these colours and patterns. Furthermore, usually it is not known whether the allele composition of the GST gene of such elite Poinsettia line is homozygous or heterozygous. For the success of the irradiation treatment it is of advantage if the bract colour of the elite Poinsettia line used as starting material is pink or marble, because these colours indicate an increased chance for heterozygous allele composition of the GST gene. In a typical experiment to generate a targeted mutation in the anthocyanin-related GST gene, the apical meristems of 30 Poinsettia young plants were irradiated with a total dosage of 30 Gray of X-ray radiation, but alternatively dosages between 15 and 50 Gray may be applied. The aim was to create single cells with a 4 bp mutation at the anthocyanin-related GST gene in one of the alleles. The irradiated Poinsettia plants were then recovered, potted and further cultivated using standard cultivation conditions in a greenhouse. The aim of the further treatment was to accumulate the mutated cell areas on mother plants grown from the irradiated Poinsettia plants. Therefore, developing shoot tips were classified as "normal" or "affected" by observation of distorted leaves. Such distortion served as marker for mutated regions. The uppermost leaf bud related to an affected leaf was identified and the shoot was pinched shortly above the identified bud using a scalpel. In case the shoot tips were completely normal, the whole side shoot was cut down to the lowest side bud. Bracts were removed that cover the centre of the plant and the lower side shoots or buds that were to develop. After pinching, the plants were grown a further 5 weeks. The newly developed side shoots with 5 to 7 leaves were then subjected to the same pinching procedure as described above. This cycle was repeated two more times. Finally resulting side shoots were harvested, rooted and potted. These plants were grown for a period of 3, 4, 5, 6, 7 or even 8 weeks in long day conditions, meaning the day length was above 12 hours. After this period the plants were grown under short day conditions with an illumination period below 12 hours per day. After a period of further 6 to 8 weeks the upper leaves modified their colour from green to either red, pink, marble, shades and variations of these colours, or the desired white foliage. The white bract, i.e. white foliage, may be only partial, further it may vary in its intensity. These plants were then selected from the plant stock and subjected to rejuvenation and further propagation with the aim to achieve a Poinsettia plant with pure white bracts and showing all the characteristics of the originating elite Poinsettia plant, except for the bract colour and some minor characteristics due to the irradiation treatment and the vegetative propagation. In case a white-bracted Poinsettia elite plant could not be obtained with a single irradiation treatment, but the mutated plants appeared to have pink or marble bracts, the irradiation treatment was repeated until the desired white-bracted genotype was found. If all the vegetative progeny of the irradiated Poinsettia elite line would show red bracts, this would indicate that the allele composition of the GST gene of the respective Poinsettia elite line is most probably homozygous for the anthocyanin-related GST wild type gene and this Poinsettia elite line is most probably not accessible to mutation to a white-bracted plant.

### Example 6: Molecular selection of heterozygous elite plant material

Cross breeding in Poinsettia aims to the creation of superior red genotypes for commercialization. Pink, marble and white sports are generated by repeated rounds of irradiation. This is however only successful in genotypes with a heterozygous allele composition in the responsible gene for foliage colouration. The allele composition of this locus is usually unknown in the art. Using the current invention, it becomes now possible to perform a molecular test to elucidate the allelic composition of the responsible EpGST gene as a codominant SSR marker. To this end, leaf material from each candidate plant was harvested. DNA was isolated from approximately 100 mg of leaf tissue using the NucleoSpin^{®} Plant II kit (Macherey-Nagel GmbH & Co. KG, Germany) according to the manufacturer's instructions. DNA was analysed for the SSR marker by Polymerase Chain Reaction (PCR) using primers
Ft (TGGCCTGCCTTTTAGAGAAA, SEQ ID No 60) and
R (ACAAGTTCAGGGGGCTGAG, SEQ ID No 10).

The PCR reactions were performed in a 20 µl reaction containing 50 ng of DNA template, 1x Williams buffer, 0.15 mM of each dNTP, 0.25 µM of primer Ft, 0.25 µM of primer R and 1u of DCSPol DNA Polymerase (DNA Cloning Service, Germany). The cycling conditions were 94°C for 3 min; 30 cycles of 94°C for 45 sec, 59°C for 1 min and 72°C for 1 min and 72°C for 1 min; and a final extension of 10 min at 72°C. The PCR products were resolved in 6% (w/v) acrylamide gel in vertical electrophoresis using the LI-COR Gene Readir 4200 DNA Analyzer (LI-COR Biosciences, Nebraska, US) (Fig 5A). The gels were subsequently stained with Ethidium bromide and the resulting band pattern was documented photographically. Alternatively, PCR products can be analysed by capillary electrophoresis on an ABI 3730 XL system at Microsynth AG (Balgach, Switzerland) (Fig. 5B).

These analyses resulted in the generation of a 195 bp DNA fragment in case of the unmutated EpGST allele, whereas the mutated allele resulted in a 191 bp DNA fragment, which could clearly be separated from the unmutated EpGST allele by the analysis methods (Fig. 5 A+B).

### Example 7: Generation of white-bracted Poinsettia elite plants from red-bracted elite Poinsettia lines with homozygous composition of the GST gene

The commercial Poinsettia elite variety Christmas Aurora (Klemm + Sohn) having a homozygous GST allele composition was chosen as starting point for targeted GST mutagenesis. The apical meristems of 30 Poinsettia young plants were irradiated with a total dosage of 15 - 50 Gray of X-ray radiation. The aim was to create single cells with a 4 bp deletion at the anthocyanin-related GST gene in one of the alleles. The irradiated Poinsettia plants were then recovered, potted and further cultivated using standard cultivation conditions in a greenhouse. The aim of the further treatment was to accumulate the mutated cell areas on mother plants growing from the irradiated Poinsettia plants. Therefore, developing shoot tips were classified as "normal" or "affected" by observation of distorted leaves. Such distortion served as marker for mutated regions. The uppermost leaf bud related to an affected leaf was identified and the shoot was pinched shortly above the identified bud using a scalpel. In case the shoot tips were completely normal, the whole side shoot was cut down to the lowest side bud. Leaves were removed that cover the centre of the plant and the lower side shoots or buds that were to develop. After pinching, the plants were grown for a further 5 weeks. The newly developed side shoots with 5 to 7 leaves were then subjected to the same pinching procedure as described above. This cycle was repeated twice. Finally, resulting side shoots were harvested, rooted and potted. These plants were subjected to marker analysis described in the above example to detect heterozygous deletion mutants of the GST gene. Among 200 recovered plants, one line showed one mutated GST allele having the expected 4 bp deletion, thus proofed to be heterozygous (Fig. 8, sample 7). Mutation frequency was confirmed to be one plant being uniformly heterozygous for the GST gene among 200 homozygous plants. The selected heterozygous plant was again subjected to the described irradiation treatment for one or several further cycles until the anthocyanin-related GST gene was homozygous for the 4 bp deletion in all tissue layers.

### Example 8: Generation of white-bracted Poinsettia elite plants through UV treatment

Ultraviolet (UV) light, in particular UVA (320 - 400 nm) and UVB (290 - 320 nm), has strong genotoxic effects to induce mutations. Commercial Poinsettia elite lines having red-coloured bracts were chosen as starting point for targeted GST mutagenesis induced by UV irradiation. In a typical experiment the apical meristems of these Poinsettia young plants were exposed to UV radiation. The aim was to create single cells with a 4 bp mutation at the anthocyanin-related GST gene in one of the alleles. After the UV treatment the irradiated Poinsettia plants were recovered, potted and further cultivated using standard cultivation conditions in a greenhouse. The aim of the further treatment was to accumulate the mutated cell areas on mother plants growing from the irradiated Poinsettia plants. The growing and selection procedures corresponded to the protocol for X-ray irradiated Poinsettia plants: Developing shoot tips were classified as "normal" or "affected" by observation of distorted leaves. Such distortion served as marker for mutated regions. The uppermost leaf bud related to an affected leaf was identified and the shoot was pinched shortly above the identified bud using a scalpel. In case the shoot tips were completely normal, the whole side shoot was cut down to the lowest side bud. Leaves were removed that cover the centre of the plant and the lower side shoots or buds that were to develop. After pinching, the plants were grown for a further 5 weeks. The newly developed side shoots with 5 to 7 leaves were then subjected to the same pinching procedure as described above. This cycle was repeated twice. Finally, resulting side shoots were harvested, rooted and potted. These plants were either selected by visual inspection or subjected to marker analysis. For visual inspection, the said plants were grown under long days for a minimum of 3 weeks followed by a period of cultivation under short days for a minimum of 6 weeks to trigger the colouration of the bracts. At this stage, white-foliaged or partially white-foliaged plants became visible and were selected. Alternatively, the propagation material from irradiated plants was subjected to marker analysis to detect deletion mutants of the GST gene. Depending on the allele composition and the chimeric status of the starting plant material white-foliaged Poinsettia elite lines could be obtained with one or more cycles of UV treatment until the anthocyanin-related GST gene was homozygous for the 4 bp mutation in all tissue layers of the elite Poinsettia plant.

### Example 9: Generation of plants with a mutant anthocyanin-related GST gene through EMS mutagenesis

Genetically stable changes in DNA sequence of plant genomes may also be induced by chemical agents. These include e.g. intercalating agents like ethidium bromide or alkylating agents like ethyl methane sulfonate (EMS). Chemical mutagenesis ideally is performed either on seeds or under aseptic conditions using tissue culture. A typical EMS mutagenesis experiment on a Poinsettia elite line starts from young shoots with minimum 4 nodal segments. After defoliation, the residual stems containing the apical meristem were surface-sterilized for 10 min. using 1.5 % (v/v) sodium hypochloride with subsequent rinsing the stems with sterile water three times. These segments were then immersed in EMS solution. The concentration of EMS varied between 0,2 - 1% depending on the respective Poinsettia genotype. The addition of 2% DMSO improved the moistening of the plant material. The incubation time varied from 30 min. up to several hours again depending on the respective Poinsettia elite line. After this treatment, the EMS solution was washed off in liquid growing media for 24 hrs, while the growing medium was exchanged twice. Then the nodal segments were transferred to growing medium according to Murashige & Skoog (MS) full-strengths medium, containing Vitamins after Nitsch, 30 g/l sucrose, 7.5 g/l plant agar and pH 5.8. No phytohormones were needed, neither for rooting nor for growth. The recovered Poinsettia plants were maintained in vitro by means of shoot cultures. The cultures were kept at 22°C under reduced light conditions (approx. 10 µmol*m⁻²*sec⁻¹) using Valoya L18 LED tubes with AP67 spectrum. Daily light period was 16 h. The further maintenance was done by sub-culturing the shoot tips and axillary nodes from in vitro plantlets using identical media and culture conditions. The aim of these propagation cycles was to accumulate the mutated cell areas on the tissue culture shoots grown from the EMS treated Poinsettia stem segments. This subculture was repeated at least twice. Then the resulting shoots were analysed for mutations in the anthocyanin-related GST gene.

### Example 10: Generation of plants with a mutant anthocyanin-related GST gene through targeted mutagenesis

Plants with white foliage are also obtainable by a targeted mutagenesis of the anthocyanin-related GST gene using gene editing approaches like CRISPR/Cas. CRISPR/Cas constructs were used to generate mutations within the coding sequences of the EpGST gene. Target sequences located on the first exon of the EpGST were designed using standard web-based CRISPR/Cas designer tools, e.g. Chopchop (https//chopchop.cbu.uib.no). Candidate sequences were synthesized (Eurofins Genomics) as oligonucleotide pairs. Each oligonucleotide carried extra bases to allow the cloning of the target sequences at the Bbsl site located between the U6-26 promoter and the sgRNA scaffold of the plant binary vector pEN-Sa-Chimera (Steinert et al., (2015) Plant J. 84:1295-1305). Resulting specific sgRNA was recombined into the destination vector pDe_Sa-cas) (http://www.botanik.kit.edu/molbio/) using the Golden Gate System following the manufacturer's instructions. All the cloning steps were done following standard procedures. Poinsettia genetic transformations were performed as described (Clarke et al., (2008) Plant Cell Rep 27(6):1027-1038) with *Agrobacterium tumefaciens* strain LBA4404 harbouring the generated CRISPR/Cas9 construct. Regenerated embryos, approximately 0.5 cm tall, were transferred to a selective rooting medium containing 2 mg/l kanamycin and grown in a climatic chamber (24-26 °C, 16 h/8 h light). Only well-rooted plantlets were transferred to the greenhouse. Plants at the stadium of having 4 - 5 expanded leaves were screened for the presence of the Cas9/sgRNA cassette by PCR using the Phire Plant Direct PCR Kit (Thermo Fisher Scientific). The amplification reactions were assembled with M13(-21) as forward primer (GTAAAACGACGGCCAG, SEQ ID No 11), common to all reactions. The reverse primer Cas9-R (CCTACAGGGAATACCTCGAGAATAT, SEQ ID No 12) was specific for the Cas9/sgRNA cassette and consisted of the same oligonucleotide employed for cloning the guide RNA. Leaf samples from T0 plants are analysed for mutations in the anthocyanin-related GST gene. Regenerated mutated Poinsettia plants were further cultivated under suitable greenhouse conditions and checked for white bracts.

## Claims

1. A method for the generation of a *Euphorbia pulcherrima (*Poinsettia*)* plant having a white foliage phenotype, comprising the steps of:
a. providing a *Euphorbia pulcherrima (*Poinsettia*)* plant without a white foliage phenotype comprising in its genome at least one functional allele of a glutathione S-transferase gene EpGST encoding a protein of SEQ ID No 3 or a functional variant of said protein with at least 90%, preferably 95% amino acid identity, the EpGST gene comprising, in the region encoding amino acids at positions 40 - 50 of the protein of SEQ ID No 3, a stretch of 12 nucleotides consisting of a threefold CTTC repeat, wherein the *Euphorbia pulcherrima (*Poinsettia*)* plant is heterozygous for the EpGST gene, comprising one functional allele as well as one dysfunctional allele comprising the CTTC deletion, said heterozygous *Euphorbia pulcherrima (*Poinsettia*)* plant without a white foliage phenotype being selected by a molecular marker suitable for the detection of the CTTC deletion;
b. subjecting the *Euphorbia pulcherrima (*Poinsettia*)* plant of step a. to a mutagenesis treatment to produce a mutant *Euphorbia pulcherrima (*Poinsettia*)* plant;
c. selecting a mutant *Euphorbia pulcherrima (*Poinsettia*)* plant of step b., wherein at least two alleles of the EpGST gene comprises a CTTC deletion in the stretch of 12 nucleotides consisting of a threefold CTTC repeat as present in the genome of the *Euphorbia pulcherrima (*Poinsettia*)* plant of step a.;
d. optionally repeating steps b. and c. until all alleles of the EpGST gene in the plant genome comprise the said CTTC deletion;
e. selecting a *Euphorbia pulcherrima (*Poinsettia*)* plant with white foliage phenotype being homozygous for the EpGST gene comprising the said CTTC deletion, and
f. optionally further asexually propagating said *Euphorbia pulcherrima (*Poinsettia*)* plant being homozygous for the EpGST gene comprising the said CTTC deletion.

2. Method of claim 1, wherein the EpGST gene is selected from the group comprising
a. genes encoding a functional homolog or variant of the protein of SEQ ID No 3 with at least 90%, preferably 95% amino acid identity, the said homolog or variant having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, the EpGST gene comprising, in the region encoding amino acids at positions 40 - 50 of the protein of SEQ ID No 3, a stretch of 12 nucleotides consisting of a threefold CTTC repeat,
b. genes encoding an mRNA corresponding to the cDNA of SEQ ID NO: 2 and functional homologs or variants of said mRNA with at least 90% nucleotide identity to the mRNA corresponding to the cDNA of SEQ ID NO: 2, the said homolog or variant comprising a stretch of 12 nucleotides consisting of a threefold CTTC repeat in the region of positions 118 - 150 of the cDNA of SEQ ID No 2,
c. the EpGST gene of SEQ ID NO: 61 and functional homologs or variants of said gene with at least 90% nucleotide identity to the gene corresponding to SEQ ID NO: 61, the said homolog or variant comprising a stretch of 12 nucleotides consisting of a threefold CTTC repeat in the region of positions 155 - 187 of the gene DNA of SEQ ID No 61,
the said functional homolog or variant of the protein of SEQ ID No 3 preferably having a V on position 2 of SEQ ID No 3, and/or an F or an L on position 62 of SEQ ID No 3, and/or LE on positions 90 - 91 of SEQ ID No 3, and/or an S on position 153 of SEQ ID No 3.

3. Method of claim 2, wherein the gene encodes
a. a functional variant or homolog of the protein of SEQ ID No 3 at least 90%, preferably 95% amino acid identity with the protein of SEQ ID No 3, preferably at least 96%, more preferably at least 97%, more preferably 98%, even more preferably at least 99%, the gene comprising, in the region encoding amino acids at positions 40 - 50 of the protein of SEQ ID No 3, a stretch of 12 nucleotides consisting of a threefold CTTC repeat;
b. a functional variant or homolog of the mRNA corresponding to the cDNA of SEQ ID No 2 having at least 95% nucleotide identity to the mRNA corresponding to SEQ ID NO: 2, preferably at least 96%, more preferably at least 97%, more preferably 98%, even more preferably at least 99%, the said homolog or variant comprising a stretch of 12 nucleotides consisting of a threefold CTTC repeat in the region of positions 118 - 150 of the cDNA of SEQ ID No 2; or
c. a functional variant or homolog of the EpGST gene of SEQ ID NO: 61having at least 95% nucleotide identity to the gene corresponding to SEQ ID NO: 61, preferably at least 96%, more preferably at least 97%, more preferably 98%, even more preferably at least 99%, the said homolog or variant comprising a stretch of 12 nucleotides consisting of a threefold CTTC repeat in the region of positions 155 - 187 of the gene DNA of SEQ ID No 61.

4. Method of any of the claims 1 to 3, wherein the EpGST cDNA has a nucleotide sequence of SEQ ID No 1.

5. Method of claim 1, wherein
a. the mutagenesis treatment is a human-induced random mutagenesis treatment,
and/or
b. the plant of step a. is genetically identical to the plant obtained in steps c. or e., apart from the CTTC deletion in the EpGST gene.

6. A white-foliaged *Euphorbia pulcherrima (*Poinsettia*)* plant, seed, plant part, plant cell, or a plant population thereof, the said plant being derived from a non-white foliaged cultivated *Euphorbia pulcherrima (*Poinsettia*)* plant, said non-white foliaged cultivated *Euphorbia pulcherrima (*Poinsettia*)* plant comprising in its genome two functional alleles of a glutathione S-transferase gene EpGST encoding a protein of SEQ ID No 3, or encoding a functional homolog or variant of said protein with at least 90%, preferably 95% amino acid identity with the said protein of SEQ ID No 3, the EpGST gene comprising, in the region encoding amino acids at positions 40 - 50 of the protein of SEQ ID No 3, a stretch of 12 nucleotides consisting of a threefold CTTC repeat; the white-foliaged derivative plant being obtained by the method of any of the preceding claims, which derivative plant is genetically identical to the non-white foliaged cultivated plant, but for
a. the presence, in each allele of the said EpGST gene a CTTC deletion in the stretch of 12 nucleotides consisting of a threefold CTTC repeat as present in the genome of the non-white foliaged cultivated plant,
b. any off-target genetic change caused by method of mutagenesis and
c. any natural genetic change during propagation,
wherein said plant, seed, plant part, plant cell, or a plant population thereof is not exclusively obtained by means of an essentially biological process.

7. A white-foliaged *Euphorbia pulcherrima (*Poinsettia*)* plant, seed, plant part, plant cell, or a plant population thereof of claim 6,
a. comprising in its genome a gene encoding a functional homolog or variant of the protein of SEQ ID No 3 with at least 90%, preferably 95% amino acid identity with the said protein of SEQ ID No 3, the said homolog or variant having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, the said gene comprising, in the region encoding amino acids at positions 40 - 50 of the protein of SEQ ID No 3, a stretch of 12 nucleotides consisting of a threefold CTTC repeat; and/or
b. comprising in each allele of the said EpGST gene a CTTC deletion in the stretch of 12 nucleotides consisting of a threefold CTTC repeat as present in the genome of the non-white foliaged cultivated *Euphorbia pulcherrima (*Poinsettia*)* plant, and
wherein said white-foliaged *Euphorbia pulcherrima (*Poinsettia*)* plant has otherwise all of the phenotypic and morphologic characteristics of the non-white foliaged *Euphorbia pulcherrima (*Poinsettia*)* plant.

8. Nucleic acid sequence of at least 20 nucleotides being complementary to the marker locus comprising the CTTC deletion within positions 128-139 of the EpGST gene of SEQ ID No 1 for detecting the presence or absence of the said sequence within the EpGST gene.

9. Use of the nucleic acid sequences of claim 8 in the selection step c. of the method of any of the claims 1 - 5.

10. Isolated genomic DNA of:
- the EpGST gene described by SEQ ID NO: 61 or
- a variant thereof having at least 90% identity to the sequence described by SEQ ID NO: 61 encoding a functional homolog or variant of the protein of SEQ ID No 3,
- the said homolog or variant having
- a first domain at positions 11 - 13 of SEQ ID No 3 being AAC, AGC or AAN, where N can be any amino acid,
- a second domain at positions 53 - 56 of SEQ ID No 3 being LVPA, QVPA or QPVP and
- a third amino acid domain at positions 65 - 68 of SEQ ID No 3 being FESR,
- the EpGST gene comprising, in the region encoding amino acids at positions 40 - 50 of the protein of SEQ ID No 3,
- a stretch of 12 nucleotides consisting of a threefold CTTC repeat, or
- the complementary sequence thereof, or
- a fragment of the gene or the complementary sequence of at least 17 contiguous nucleotides,
the said isolated DNA of the EpGST gene, the complementary sequence thereof, or a fragment of the gene or the complementary sequence of at least 17 contiguous nucleotides comprising a CTTC deletion as identified in any of the preceding claims within positions 128-139 of SEQ ID No 1.

11. Isolated cDNA encoding a EpGST protein described by SEQ ID NO: 3 or encoding a functional homolog or variant of said protein with at least 90%, preferably 95% amino acid identity, the said homolog or variant having a first domain at positions 11 - 13 of SEQ ID No 3 being AAC, AGC or AAN, where N can be any amino acid, a second domain at positions 53 - 56 of SEQ ID No 3 being LVPA, QVPA or QPVP and a third amino acid domain at positions 65 - 68 of SEQ ID No 3 being FESR, or the complements thereof.

12. A method of use of the isolated DNA selected from the group consisting of (i) the sequences of any of claim 10 or 11 and (ii) the sequences of SEQ ID NO: 44 to 49, or variants with at least 95% identity therewith for the preparation of a nucleic acid sequence of claim 8 or for a method for targeted mutagenesis of a GST gene in a target plant, preferably using a continuous stretch of at least 17 nucleotides from any said isolated DNA sequences to (a) produce a guide RNA or an expression construct therefor for a CRISPR/Cas-based method of gene editing or (b) produce a silencing RNA or an expression construct therefor for a method of RNA-mediated gene silencing.

## Patentansprüche

1. Verfahren zur Erzeugung einer *Euphorbia pulcherrima* (Poinsettia)-Pflanze mit weißem Laub-Phänotyp, umfassend die folgenden Schritte:
a. Bereitstellen einer *Euphorbia pulcherrima* (Poinsettia)-Pflanze ohne Weißblatt-Phänotyp, die in ihrem Genom mindestens ein funktionelles Allel eines Glutathion-S-Transferase-Gens EpGST umfasst, das für ein Protein der SEQ ID Nr. 3 oder eine funktionelle Variante dieses Proteins mit mindestens 90 %, vorzugsweise 95 %, Aminosäure-Identität kodiert, wobei das EpGST-Gen in der Region, die für die Aminosäuren an den Positionen 40 bis 50 des Proteins der SEQ ID Nr. 3 kodiert, einen Abschnitt von 12 Nukleinsäuren umfasst, der aus einer dreifachen CTTC-Wiederholung besteht, wobei die *Euphorbia pulcherrima* (Poinsettia)-Pflanze heterozygot für das EpGST-Gen ist und sowohl ein funktionelles Allel als auch ein dysfunktionelles Allel umfasst, das die CTTC-Deletion umfasst, wobei die heterozygote *Euphorbia pulcherrima* (Poinsettia)-Pflanze ohne einen Phänotyp mit weißem Laub durch einen molekularen Marker selektiert wird, der für den Nachweis der CTTC-Deletion geeignet ist;
b. Unterziehen der *Euphorbia pulcherrima* (Poinsettia)-Pflanze aus Schritt a. einer Mutagenesebehandlung, um eine mutierte *Euphorbia pulcherrima* (Poinsettia)-Pflanze zu erzeugen;
c. Auswahl einer mutierten *Euphorbia pulcherrima* (Poinsettia)-Pflanze aus Schritt b., wobei mindestens zwei Allele des EpGST-Gens eine CTTC-Deletion in dem Abschnitt von 12 Nukleotiden umfassen, der aus einer dreifachen CTTC-Wiederholung besteht, wie sie im Genom der *Euphorbia pulcherrima* (Poinsettia)-Pflanze aus Schritt a. vorhanden ist;
d. gegebenenfalls Wiederholung der Schritte b. und c., bis alle Allele des EpGST-Gens im Pflanzengenom die genannte CTTC-Deletion aufweisen;
e. Selektion einer *Euphorbia pulcherrima* (Poinsettia)-Pflanze mit dem Phänotyp des weißen Laubs, die homozygot für das EpGST-Gen ist, das die CTTC-Deletion aufweist, und
f. gegebenenfalls weitere asexuelle Vermehrung der *Euphorbia pulcherrima* (Poinsettia)-Pflanze, die homozygot für das EpGST-Gen mit der CTTC-Deletion ist.

2. Verfahren nach Anspruch 1, wobei das EpGST-Gen ausgewählt ist aus der Gruppe, die umfasst
a. Gene, die für ein funktionelles Homolog oder eine Variante des Proteins der SEQ ID Nr. 3 mit mindestens 90 %, vorzugsweise 95 % Aminosäureidentität kodieren, wobei das Homolog oder die Variante eine erste Domäne an den Positionen 11 - 13 der SEQ ID Nr. 3 aufweist, die AAC, AGC oder AAN ist, wobei N eine beliebige Aminosäure sein kann, eine zweite Domäne an den Positionen 53 - 56 der SEQ ID Nr. 3 LVPA, QVPA oder QPVP ist und eine dritte Aminosäuredomäne an den Positionen 65 - 68 von SEQ ID Nr. 3 FESR ist, wobei das EpGST-Gen in der Region, die für die Aminosäuren an den Positionen 40 - 50 des Proteins von SEQ ID Nr. 3 kodiert, einen Abschnitt von 12 Nukleotiden umfasst, der aus einer dreifachen CTTC-Wiederholung besteht,
b. Gene, die für eine mRNA kodieren, die der cDNA von SEQ ID NO: 2 entspricht, und funktionelle Homologe oder Varianten dieser mRNA mit mindestens 90 % Nukleotididentität mit der mRNA, die der cDNA von SEQ ID NO: 2 entspricht, wobei das Homolog oder die Variante einen Abschnitt von 12 Nukleotiden umfasst, der aus einer dreifachen CTTC-Wiederholung in der Region der Positionen 118 - 150 der cDNA von SEQ ID NO: 2 besteht,
c. das EpGST-Gen der SEQ ID NO: 61 und funktionelle Homologe oder Varianten dieses Gens mit mindestens 90 % Nukleotididentität mit dem Gen, das der SEQ ID NO: 61 entspricht, wobei das Homolog oder die Variante einen Abschnitt von 12 Nukleotiden umfasst, der aus einer dreifachen CTTC-Wiederholung in der Region der Positionen 155 - 187 der Gen-DNA der SEQ ID NO: 61 besteht,
wobei das funktionelle Homolog oder die Variante des Proteins von SEQ ID Nr. 3 vorzugsweise ein V an Position 2 von SEQ ID Nr. 3 und/oder ein F oder ein L an Position 62 von SEQ ID Nr. 3 und/oder LE an den Positionen 90 - 91 von SEQ ID Nr. 3 und/oder ein S an Position 153 von SEQ ID Nr. 3 aufweist.

3. Verfahren nach Anspruch 2, wobei das Gen für Folgendes kodiert
a. eine funktionelle Variante oder ein Homolog des Proteins von SEQ ID Nr. 3 mit mindestens 90 %, vorzugsweise 95 % Aminosäure-Identität mit dem Protein von SEQ ID Nr. 3, vorzugsweise mindestens 96 %, noch bevorzugter mindestens 97 %, noch bevorzugter 98 %, noch bevorzugter mindestens 99 %, wobei das Gen in der Region, die für die Aminosäuren an den Positionen 40 - 50 des Proteins von SEQ ID Nr. 3 kodiert, einen Abschnitt von 12 Nukleotiden umfasst, der aus einer dreifachen CTTC-Wiederholung besteht;
b. eine funktionelle Variante oder ein Homolog der mRNA, die der cDNA von SEQ ID Nr. 2 entspricht, mit mindestens 95 % Nukleotididentität mit der mRNA, die SEQ ID Nr. 2 entspricht, vorzugsweise mindestens 96 %, noch bevorzugter mindestens 97 %, noch bevorzugter 98 %, noch bevorzugter mindestens 99 %, wobei das Homolog oder die Variante einen Abschnitt von 12 Nukleotiden umfasst, der aus einer dreifachen CTTC-Wiederholung in der Region der Positionen 118 - 150 der cDNA von SEQ ID Nr. 2 besteht;
oder
c. eine funktionelle Variante oder ein Homolog des EpGST-Gens der SEQ ID Nr. 61 mit mindestens 95 % Nukleotididentität mit dem Gen, das der SEQ ID Nr. 61 entspricht, vorzugsweise mindestens 96 %, noch bevorzugter mindestens 97 %, noch bevorzugter 98 %, noch bevorzugter mindestens 99 %, wobei das Homolog oder die Variante einen Abschnitt von 12 Nukleotiden umfasst, der aus einer dreifachen CTTC-Wiederholung in der Region der Positionen 155 - 187 der Gen-DNA der SEQ ID Nr. 61 besteht.

4. Verfahren nach einem der Ansprüche 1 bis 3, wobei die EpGST-cDNA die Nukleotidsequenz von SEQ ID Nr. 1 aufweist.

5. Verfahren nach Anspruch 1, wobei
a. die Mutagenese-Behandlung eine vom Menschen induzierte Zufallsmutagenese-Behandlung ist, und/oder
b. die Pflanze aus Schritt a. genetisch identisch mit der in den Schritten c. oder e. erhaltenen Pflanze ist, abgesehen von der CTTC-Deletion im EpGST-Gen.

6. Weißblättrige *Euphorbia pulcherrima* (Poinsettia)-Pflanze, -Samen, -Pflanzenteil, - Pflanzenzelle oder eine Pflanzenpopulation davon, wobei die Pflanze von einer nicht-weißblättrigen kultivierten *Euphorbia pulcherrima* (Poinsettia)-Pflanze abgeleitet ist, wobei die nicht-weißblättrige kultivierte *Euphorbia pulcherrima* (Poinsettia)-Pflanze in ihrem Genom zwei funktionelle Allele eines Glutathion-S-Transferase-Gens EpGST umfasst, das für ein Protein der SEQ ID Nr. 3 kodiert, oder ein funktionelles Homolog oder eine Variante dieses Proteins mit mindestens 90 %, vorzugsweise 95 %, Aminosäureidentität mit dem Protein der SEQ ID Nr. 3 kodiert, wobei das EpGST-Gen in der Region, die für die Aminosäuren an den Positionen 40 bis 50 des Proteins der SEQ ID Nr. 3 kodiert, einen Abschnitt von 12 Nukleotiden umfasst, der aus einer dreifachen CTTC-Wiederholung besteht; die weißblättrige Derivatpflanze, die durch das Verfahren nach einem der vorhergehenden Ansprüche erhalten wird, wobei die Derivatpflanze genetisch identisch mit der nicht-weißblättrigen Kulturpflanze ist, mit Ausnahme von
a. das Vorhandensein einer CTTC-Deletion in jedem Allel des EpGST-Gens in dem Abschnitt von 12 Nukleotiden, der aus einer dreifachen CTTC-Wiederholung besteht, wie sie im Genom der nicht-weiß belaubten Kulturpflanze vorhanden ist,
b. jede durch eine Mutagenesemethode verursachte genetische Veränderung außerhalb des Ziels und
c. jede natürliche genetische Veränderung während der Vermehrung,
wobei die Pflanze, das Saatgut, der Pflanzenteil, die Pflanzenzelle oder eine Pflanzenpopulation davon nicht ausschließlich durch ein im Wesentlichen biologisches Verfahren gewonnen wird.

7. Weißblättrige *Euphorbia pulcherrima* (Poinsettia)-Pflanze, -Samen, -Pflanzenteil, - Pflanzenzelle oder eine Pflanzenpopulation davon nach Anspruch 6,
a. die in ihrem Genom ein Gen enthält, das für ein funktionelles Homolog oder eine Variante des Proteins der SEQ ID Nr. 3 mit mindestens 90 %, vorzugsweise 95 % Aminosäureidentität mit dem Protein der SEQ ID Nr. 3 kodiert, wobei das Homolog oder die Variante eine erste Domäne an den Positionen 11 - 13 der SEQ ID Nr. 3 aufweist, die AAC, AGC oder AAN ist, wobei N eine beliebige Aminosäure sein kann, wobei N eine beliebige Aminosäure sein kann, eine zweite Domäne an den Positionen 53 - 56 der SEQ ID Nr. 3 LVPA, QVPA oder QPVP ist und eine dritte Aminosäuredomäne an den Positionen 65 - 68 der SEQ ID Nr. 3 FESR ist, wobei das Gen in der Region, die für Aminosäuren an den Positionen 40 - 50 des Proteins der SEQ ID Nr. 3 kodiert, einen Abschnitt von 12 Nukleotiden umfasst, der aus einer dreifachen CTTC-Wiederholung besteht; und/oder
b. in jedem Allel des EpGST-Gens eine CTTC-Deletion in dem aus einer dreifachen CTTC-Wiederholung bestehenden Abschnitt von 12 Nukleotiden, wie er im Genom der nicht-weißblättrigen Kulturpflanze *Euphorbia pulcherrima* (Poinsettia) vorhanden ist, aufweist und wobei die weißblättrige *Euphorbia pulcherrima* (Poinsettia)-Pflanze ansonsten alle phänotypischen und morphologischen Merkmale der nicht-weißblättrigen *Euphorbia pulcherrima* (Poinsettia)-Pflanze aufweist.

8. Nukleinsäuresequenz mit mindestens 20 Nukleotiden, die komplementär zu dem Markerlocus ist, der die CTTC-Deletion innerhalb der Positionen 128-139 des EpGST-Gens von SEQ ID Nr. 1 umfasst, um das Vorhandensein oder Fehlen der Sequenz innerhalb des EpGST-Gens nachzuweisen.

9. Verwendung der Nukleinsäuresequenzen nach Anspruch 8 im Selektionsschritt c. des Verfahrens nach einem der Ansprüche 1 bis 5.

10. Isolierte genomische DNA von:
- dem EpGST-Gen, beschrieben durch SEQ ID NO: 61 oder
- einer Variante davon mit mindestens 90 % Identität mit der durch SEQ ID NO: 61 beschriebenen Sequenz, die ein funktionelles Homolog oder eine Variante des Proteins von SEQ ID NO: 3 codiert,
- wobei das Homolog oder die Variante Folgendes aufweist
- eine erste Domäne an den Positionen 11 - 13 der SEQ ID Nr. 3, die AAC, AGC oder AAN ist, wobei N eine beliebige Aminosäure sein kann,
- eine zweite Domäne an den Positionen 53 - 56 von SEQ ID Nr. 3, die LVPA, QVPA oder QPVP ist, und
- eine dritte Aminosäuredomäne an den Positionen 65 - 68 der SEQ ID Nr. 3, die FESR ist,
- das EpGST-Gen, das in der Region, die für die Aminosäuren an den Positionen 40 - 50 des Proteins der SEQ ID Nr. 3 kodiert
- einen Abschnitt von 12 Nukleotiden, bestehend aus einer dreifachen CTTC-Wiederholung, oder
- die komplementäre Sequenz davon, oder
- ein Fragment des Gens oder die komplementäre Sequenz von mindestens 17 zusammenhängenden Nukleotiden,
die isolierte DNA des EpGST-Gens, die komplementäre Sequenz davon oder ein Fragment des Gens oder die komplementäre Sequenz von mindestens 17 zusammenhängenden Nukleotiden, die eine CTTC-Deletion, wie sie in einem der vorhergehenden Ansprüche identifiziert wurde, innerhalb der Positionen 128-139 von SEQ ID Nr. 1 umfasst.

11. Isolierte cDNA, die für ein EpGST-Protein, beschrieben durch SEQ ID NO: 3 beschrieben ist, oder für ein funktionelles Homolog oder eine Variante des Proteins mit mindestens 90 %, vorzugsweise 95 %, Aminosäureidentität kodiert, wobei das Homolog oder die Variante eine erste Domäne an den Positionen 11 - 13 der SEQ ID Nr. 3, die AAC, AGC oder AAN ist, wobei N eine beliebige Aminosäure sein kann, eine zweite Domäne an den Positionen 53 - 56 der SEQ ID Nr. 3, die LVPA, QVPA oder QPVP ist, und eine dritte Aminosäuredomäne an den Positionen 65 - 68 der SEQ ID Nr. 3, die FESR ist, oder die Komplemente davon aufweist.

12. Verfahren zur Verwendung der isolierten DNA, ausgewählt aus der Gruppe bestehend aus (i) den Sequenzen nach einem der Ansprüche 10 oder 11 und (ii) den Sequenzen von SEQ ID NO: 44 bis 49, oder Varianten mit mindestens 95 % Identität damit, zur Herstellung einer Nukleinsäuresequenz nach Anspruch 8 oder für ein Verfahren zur gezielten Mutagenese eines GST-Gens in einer Zielpflanze, vorzugsweise unter Verwendung eines kontinuierlichen Abschnitts von mindestens 17 Nukleotiden aus einer der isolierten DNA-Sequenzen, um (a) eine Leit-RNA oder ein Expressionskonstrukt dafür für ein CRISPR/Cas-basiertes Verfahren zum Gen-Editing herzustellen oder (b) eine Silencing-RNA oder ein Expressionskonstrukt dafür für ein Verfahren zum RNA-vermittelten Gen-Silencing herzustellen.

## Revendications

1. Méthode de génération d'une plante *Euphorbia pulcherrima* (Poinsettia) présentant un phénotype de feuillage blanc, comprenant les étapes suivantes
a. fournir une plante *Euphorbia pulcherrima* (Poinsettia) sans phénotype de feuillage blanc comprenant dans son génome au moins un allèle fonctionnel d'un gène de glutathion S-transférase EpGST codant une protéine de SEQ ID No 3 ou une variante fonctionnelle de ladite protéine avec au moins 90%, de préférence 95% d'identité d'acide aminé, le gène EpGST comprenant, dans la région codant les acides aminés aux positions 40 - 50 de la protéine de SEQ ID No 3, un tronçon de 12 nucléotides consistant en une triple répétition CTTC, dans lequel la plante *Euphorbia pulcherrima* (Poinsettia) est hétérozygote pour le gène EpGST, comprenant un allèle fonctionnel ainsi qu'un allèle dysfonctionnel comprenant la délétion CTTC, ladite plante *Euphorbia pulcherrima* (Poinsettia) hétérozygote sans phénotype de feuillage blanc étant sélectionnée par un marqueur moléculaire adapté à la détection de la délétion CTTC ;
b. soumettre la plante *Euphorbia pulcherrima* (Poinsettia) de l'étape a. à un traitement de mutagenèse pour produire une plante *Euphorbia pulcherrima* (Poinsettia) mutante ;
c. sélection d'une plante *Euphorbia pulcherrima* (Poinsettia) mutante de l'étape b., dans laquelle au moins deux allèles du gène EpGST comprennent une délétion CTTC dans la portion de 12 nucléotides consistant en une triple répétition CTTC telle qu'elle est présente dans le génome de la plante *Euphorbia pulcherrima* (Poinsettia) de l'étape a. ;
d. en répétant éventuellement les étapes b. et c. jusqu'à ce que tous les allèles du gène EpGST dans le génome de la plante comprennent ladite délétion CTTC ;
e. sélection d'une plante *Euphorbia pulcherrima* (Poinsettia) à phénotype de feuillage blanc homozygote pour le gène EpGST comprenant ladite délétion CTTC, et
f. éventuellement, propagation asexuée de ladite plante *Euphorbia pulcherrima* (Poinsettia) homozygote pour le gène EpGST comprenant ladite délétion CTTC.

2. Méthode de la revendication 1, dans laquelle le gène EpGST est choisi dans le groupe comprenant
a. les gènes codant pour un homologue fonctionnel ou un variant de la protéine de SEQ ID No 3 avec au moins 90%, de préférence 95% d'identité d'acide aminé, ledit homologue ou variant ayant un premier domaine aux positions 11 - 13 de SEQ ID No 3 étant AAC, AGC ou AAN, où N peut être n'importe quel acide aminé, un second domaine aux positions 53 - 56 de SEQ ID No 3 étant LVPA, QVPA ou QPVP et un troisième domaine d'acide aminé aux positions 65 - 68 de SEQ ID n° 3 étant FESR, le gène EpGST comprenant, dans la région codant les acides aminés aux positions 40 - 50 de la protéine de SEQ ID n° 3, un tronçon de 12 nucléotides consistant en une triple répétition CTTC,
b. les gènes codant pour un ARNm correspondant à l'ADNc de SEQ ID NO : 2 et les homologues fonctionnels ou les variantes dudit ARNm ayant au moins 90 % d'identité nucléotidique avec l'ARNm correspondant à l'ADNc de SEQ ID NO : 2, ledit homologue ou variante comprenant un tronçon de 12 nucléotides consistant en une triple répétition CTTC dans la région des positions 118 - 150 de l'ADNc de SEQ ID NO : 2,
c. le gène EpGST de SEQ ID NO : 61 et les homologues ou variants fonctionnels dudit gène présentant une identité nucléotidique d'au moins 90 % avec le gène correspondant à SEQ ID NO : 61, ledit homologue ou variant comprenant un tronçon de 12 nucléotides consistant en une triple répétition CTTC dans la région des positions 155 - 187 de l'ADN du gène de SEQ ID NO : 61,
ledit homologue fonctionnel ou variante de la protéine de SEQ ID n° 3 ayant de préférence un V en position 2 de SEQ ID n° 3, et/ou un F ou un L en position 62 de SEQ ID n° 3, et/ou un LE en positions 90 - 91 de SEQ ID n° 3, et/ou un S en position 153 de SEQ ID n° 3.

3. Méthode de la revendication 2, dans laquelle le gène code pour
a. un variant fonctionnel ou un homologue de la protéine SEQ ID n° 3 ayant au moins 90 %, de préférence 95 % d'identité d'acide aminé avec la protéine SEQ ID n° 3, de préférence au moins 96 %, de préférence au moins 97 %, de préférence 98 %, de préférence encore au moins 99 %, le gène comprenant, dans la région codant pour les acides aminés aux positions 40 - 50 de la protéine SEQ ID n° 3, un tronçon de 12 nucléotides consistant en une triple répétition CTTC ;
b. un variant fonctionnel ou un homologue de l'ARNm correspondant à l'ADNc de SEQ ID n° 2 ayant au moins 95 % d'identité nucléotidique avec l'ARNm correspondant à SEQ ID n° 2, de préférence au moins 96 %, de préférence au moins 97 %, de préférence 98 %, de préférence encore au moins 99 %, ledit homologue ou variant comprenant un segment de 12 nucléotides constitué d'une triple répétition CTTC dans la région des positions 118 - 150 de l'ADNc de SEQ ID n° 2 ; ou
c. un variant fonctionnel ou un homologue du gène EpGST de SEQ ID NO : 61 ayant au moins 95 % d'identité nucléotidique avec le gène correspondant à SEQ ID NO : 61, de préférence au moins 96 %, de préférence au moins 97 %, de préférence 98 %, de préférence encore au moins 99 %, ledit homologue ou variant comprenant un tronçon de 12 nucléotides consistant en une triple répétition CTTC dans la région des positions 155 - 187 de l'ADN du gène de SEQ ID NO : 61.

4. Méthode de l'une des revendications 1 à 3, dans laquelle l'ADNc EpGST a une séquence nucléotidique de SEQ ID No 1.

5. Méthode de la revendication 1, dans laquelle
a. le traitement de mutagenèse est un traitement de mutagenèse aléatoire induite par l'homme, et/ou
b. la plante de l'étape a. est génétiquement identique à la plante obtenue aux étapes c. ou e., à l'exception de la délétion CTTC dans le gène EpGST.

6. Plante, graine, partie de plante, cellule végétale ou population végétale d'*Euphorbia pulcherrima* (Poinsettia) à feuillage blanc, ladite plante étant dérivée d'une plante *Euphorbia pulcherrima* (Poinsettia) cultivée à feuillage non blanc, ladite plante *Euphorbia pulcherrima* (Poinsettia) cultivée à feuillage non blanc comprenant dans son génome deux allèles fonctionnels d'un gène de glutathion S-transférase EpGST codant pour une protéine de SEQ ID No 3, ou codant un homologue fonctionnel ou une variante de ladite protéine présentant une identité d'acide aminé d'au moins 90 %, de préférence 95 %, avec ladite protéine de SEQ ID n° 3, le gène EpGST comprenant, dans la région codant les acides aminés aux positions 40 - 50 de la protéine de SEQ ID n° 3, un tronçon de 12 nucléotides consistant en une triple répétition CTTC ; la plante dérivée à feuillage blanc obtenue par le procédé de l'une quelconque des revendications précédentes, laquelle plante dérivée est génétiquement identique à la plante cultivée à feuillage non blanc, à l'exception de
a. la présence, dans chaque allèle dudit gène EpGST, d'une délétion CTTC dans la bande de 12 nucléotides consistant en une triple répétition CTTC telle qu'elle est présente dans le génome de la plante cultivée à feuillage non blanc,
b. toute modification génétique hors cible causée par une méthode de mutagenèse et
c. toute modification génétique naturelle au cours de la propagation,
dans lequel ladite plante, graine, partie de plante, cellule végétale ou une population végétale de celle-ci n'est pas exclusivement obtenue au moyen d'un procédé essentiellement biologique.

7. Plante, graine, partie de plante, cellule végétale ou population végétale d'*Euphorbia pulcherrima* (Poinsettia) à feuillage blanc selon la revendication 6,
a. comprenant dans son génome un gène codant pour un homologue fonctionnel ou une variante de la protéine de SEQ ID No 3 avec au moins 90%, de préférence 95% d'identité d'acide aminé avec ladite protéine de SEQ ID No 3, ledit homologue ou variante ayant un premier domaine aux positions 11 - 13 de SEQ ID No 3 étant AAC, AGC ou AAN, où N peut être n'importe quel acide aminé, un deuxième domaine aux positions 53 - 56 de SEQ ID No 3 étant LVPA, QVPA ou QPVP et un troisième domaine d'acide aminé aux positions 65 - 68 de SEQ ID No 3 étant FESR, ledit gène comprenant, dans la région codant pour les acides aminés aux positions 40 - 50 de la protéine de SEQ ID No 3, un tronçon de 12 nucléotides consistant en une triple répétition CTTC ; et/ou
b. comprenant dans chaque allèle dudit gène EpGST une délétion CTTC dans la portion de 12 nucléotides consistant en une triple répétition CTTC telle qu'elle est présente dans le génome de la plante cultivée *Euphorbia pulcherrima* (Poinsettia) à feuillage non blanc, et dans laquelle ladite plante *Euphorbia pulcherrima* (Poinsettia) à feuillage blanc présente par ailleurs toutes les caractéristiques phénotypiques et morphologiques de la plante *Euphorbia pulcherrima* (Poinsettia) à feuillage non blanc.

8. Séquence d'acide nucléique d'au moins 20 nucléotides complémentaire au locus marqueur comprenant la délétion CTTC à l'intérieur des positions 128-139 du gène EpGST de SEQ ID No 1 pour détecter la présence ou l'absence de ladite séquence à l'intérieur du gène EpGST.

9. Utilisation des séquences d'acides nucléiques de la revendication 8 dans l'étape de sélection c. de la méthode de l'une quelconque des revendications 1 à 5.

10. ADN génomique isolé du :
- le gène EpGST décrit par SEQ ID NO : 61 ou
- un variant de celui-ci ayant au moins 90% d'identité avec la séquence décrite par SEQ ID NO : 61 codant un homologue fonctionnel ou un variant de la protéine de SEQ ID No 3,
- ledit homologue ou variant ayant
- un premier domaine aux positions 11 - 13 de SEQ ID n° 3 étant AAC, AGC ou AAN, où N peut être n'importe quel acide aminé,
- un deuxième domaine aux positions 53 - 56 de SEQ ID No 3 étant LVPA, QVPA ou QPVP et
- un troisième domaine d'acide aminé aux positions 65 - 68 de SEQ ID No 3 étant FESR,
- le gène EpGST comprenant, dans la région codant les acides aminés aux positions 40 - 50 de la protéine de SEQ ID No 3,
- un tronçon de 12 nucléotides consistant en une triple répétition CTTC, ou
- sa séquence complémentaire, ou
- un fragment du gène ou de la séquence complémentaire d'au moins 17 nucléotides contigus,
ledit ADN isolé du gène EpGST, sa séquence complémentaire ou un fragment du gène ou de la séquence complémentaire d'au moins 17 nucléotides contigus comprenant une délétion CTTC telle qu'identifiée dans l'une des revendications précédentes à l'intérieur des positions 128-139 de SEQ ID No 1.

11. ADNc isolé codant pour une protéine EpGST décrite par SEQ ID NO : 3 ou codant un homologue fonctionnel ou un variant de ladite protéine avec au moins 90%, de préférence 95% d'identité d'acide aminé, ledit homologue ou variant ayant un premier domaine aux positions 11 - 13 de SEQ ID No 3 étant AAC, AGC ou AAN, où N peut être n'importe quel acide aminé, un deuxième domaine aux positions 53 - 56 de SEQ ID No 3 étant LVPA, QVPA ou QPVP et un troisième domaine d'acide aminé aux positions 65 - 68 de SEQ ID No 3 étant FESR, ou les compléments de celui-ci.

12. Méthode d'utilisation de l'ADN isolé choisi dans le groupe constitué (i) des séquences de l'une des revendications 10 ou 11 et (ii) des séquences de SEQ ID NO : 44 à 49, ou des variantes ayant au moins 95% d'identité avec celles-ci, pour la préparation d'une séquence d'acide nucléique de la revendication 8 ou pour une méthode de mutagenèse ciblée d'un gène GST dans une plante cible, de préférence en utilisant un tronçon continu d'au moins 17 nucléotides de ces séquences d'ADN isolées pour (a) produire un ARN guide ou une construction d'expression pour une méthode d'édition de gènes basée sur CRISPR/Cas ou (b) produire un ARN de silencieux ou une construction d'expression pour une méthode de silencieux de gènes médiée par l'ARN.
